# EUROPEAN PATENT SPECIFICATION

(11) **EP 0 381 617 B1**
(45) Date of publication and mention of the grant of the patent: **15.05.1996**
(21) Application number: 90810048.0
(22) Date of filing: 23.01.1990
(51) Int. Cl.: A61L 2/00, C11D 3/00, C11D 3/20

(54) **Disinfecting and cleaning composition for contact lenses**
Desinfektions- und Reinigungsmittel für Kontaktlinsen
Composition désinfectante et nettoyante pour lentilles de contact

(30) Priority: 31.01.1989 US 304746
(43) Date of publication of application: 08.08.1990
(73) Proprietor: CIBA-GEIGY AG, CH-4002 Basel (CH)
(72) Inventor: Tsao, Fu-Pao, Lawrenceville, GA 30245 (US); Littlefield, Susan Ann, Norcross, GA 30071 (US); Stone, John Harlan, Conyers, GA 30208 (US)

(56) References cited:
- EP-A- 0 001 888
- EP-A- 0 209 192
- EP-A- 0 233 842
- EP-A- 0 358 447
- GB-A- 2 103 642
- US-A- 3 367 878
- US-A- 4 510 065

## Description

The invention is in the area of contact lens care products, particularly solutions used to clean and disinfect contact lenses.

Disinfection solutions for use in conjunction with contact lenses have been in use essentially as long as contact lenses have been available to the public. There is a large diversity in the makeup of the various known solutions, primarily due to the fact that to date no single solution has been found to meet all of the parameters desired in a single solution. For example, currently commercially available solutions such as ReNu Disinfectant (Bausch & Lomb), Optisoft (Alcon), and Optifree (Alcon) which offer low irritancy and/or hypersensitivity require a minimum of four hours soaking to disinfect. Solutions such as Flexcare contain thimerosol which has been particularly problematical as a disinfecting or preservative agent. Because of these problems there has been an attempt to avoid thimerosol as an antimicrobial agent.

A second, and not small consideration, is contact lens material/solution compatibility. Heat disinfection is not a practical alternative for use with high water content soft contact lenses. Some lenses entrap or react with various components of the disinfection solution making it impossible to utilize such solutions with those lenses. For this reason, proper patient compliance with lens/solution match-up directions is essential to maintaining contact lenses properly. Yet experience has shown that patient compliance with lens and solution manufacturer directions is not adhered to by a significant, although small patient population. Hence, there has been an effort to develop a disinfection solution which is generally useful for most, if not all contact lenses currently available.

Finally, not all disinfectant solutions are suitably effective against the entire range of microbial organisms which are of concern in the contact lens field. One such organism where disinfectants and preservatives have had limited success is Acanthamoeba. The present invention solution is effective against the cyst as well as the trophozite stage of these protozoa.

EP A 358 447 discloses a cleaning, conditioning, storing and wetting system for contact lenses using a cleaning, conditioning and storing solution and a separate wetting solution each containing high purity benzyl alcohol or sorbic acid. The wetting solution preferably has a tonicity of from about 0.91 to about 1.65, while sodium chloride can be present in the solution in an amount of up to about 2.0 %. Higher tonicities are not disclosed.

US A 4,510,065 discloses a contact lens preservative system and a prophylactic cleaner comprising trimethoprim and adjuvant bactericides including EDTA or a water soluble salt thereof and sorbic acid. While these solutions may be mildly hypertonic, use of high concentrations of sodium chloride is not recommended since such method is harmful to the lenses because scratching of the lens surface occurs. Therefore, the recommended tonicity of the aqueous cleaning composition is from about 1.0 to about 2.0.

EP A 001 888 discloses an aqueous sterilizing solution comprising a physiologically acceptable salt in a concentration of at least 5 % w/w, which is preferably sodium chloride, a physiologically acceptable alcohol, which is preferably ethanol, and a water-soluble, physiologically acceptable, protein-denaturing agent, which is preferably urea. This solution permits rapid sterilization of e.g. contact lenses. However, the rapid sterilization is disclosed to last at least 30 minutes.

US A 3,367,878 discloses alkaline water based cleaners comprising a number of ingredients designed to clean aircraft surfaces by immersion, spraying, brushing or preferably by a hydrosteam cleaning machine. These cleaners comprise as low amounts of surfactants as 0.34 % to 1.5 %.

In view of the prior art mentioned hereinbefore and in view of the products marketed at present there is still the need for an effective and rapid cleaning and disinfecting solution, especially such solution for contact lenses.

One object of the invention is to provide a contact lens disinfecting solution which will be non-irritating to the patient after following a simple, easy to carry out disinfecting regimen.

A second object of the invention is to provide a one step cleaning and disinfecting solution meeting the foregoing object.

A third object is to provide a contact lens disinfection solution having compatibility with essentially all currently available contact lenses.

A fourth object of the invention is to provide a disinfection solution which is effective against a wide range of ocular pathogens including Acanthamoeba.

Surprisingly, the foregoing objects and others are achieved by a solution for cleaning and disinfecting contact lenses comprising
a) x % by weight of a C₃₋₈alkylene glycol, and
   y% by weight of a C₂₋₆alkanol
   wherein x is zero to 50 and y is zero to 30 with the proviso that $\text{x/10 + y/2 ≧ 1.0}$;
b) an amount of a tonicity builder sufficient to raise the solution tonicity to the equivalent of a 5 to 20 % by weight sodium chloride solution;
c) 2 to 25 % by weight of an ophthalmic device material compatible surfactant;
d) 0 to 2 % by weight of a pH adjusting or regulating agent;
e) 0 % to an amount sufficient to bring the solution viscosity to 100 cps of a viscosity enhancing agent; and
f) the balance of a suitable solvent which is preferably water.

Preferred embodiments of the invention are disclosed in claims 2 to 15.

It should be emphasized here that the invention is also applicable beyond the contact lens disinfection and preservative field and may be used anywhere a disinfecting solution treatment or preserved solution would be useful provided only that the subject material to be treated is not adversely affected by the solution components. For these purposes, the invention solution need not be contact lens compatible or even pharmaceutically acceptable. Typical non-contant lens disinfecting applications for which such solutions are useful include: lens case cleaner and disinfectant, topical medical composition, cosmetic, facial cleaner, hand cleaner, disinfecting soaps such as surgical soap, shampoo, household disinfectant, and industrial disinfectant, laboratory disinfectant, dental and medical equipment disinfectant, acne cleaning and disinfecting treatments, insect bite disinfection, for minor skin itching and rashes and wound healing applications. It is also suitable as a rapid in-office contact lens disinfecting/cleaning regimen.

Other components may also be present which are typical for the type of formulation useful for the purpose to which the inventive solution is being put. Hence if the solution is to be a cleanser where surface scratching is not of concern, agents such as silicon dioxide may be present as well.

The formulations of the invention are typically used by contacting the surface to be treated with the formulations, rubbing up the formulation on the surface from 5-30 seconds and rinsing the treated surface. In terms of the preferred use, contact lens disinfection, the solution is placed on the lens in the same manner as any other cleaner or disinfectant for contact lenses, rubbed lightly for 5-30 seconds and rinsed with water or normal saline as appropriate.

In the typical formulation of the invention, the C₃₋₈alkylene glycol is present from 10 % to 50 % by weight, preferably 15 % to 40 % by weight, still more preferably 17 % to 25 % by weight, most preferably 21 % by weight of the entire formulation.

The C₃₋₈alkylene glycol is preferably selected from 1,2 or alpha,omega glycols such as 1,2-propylene glycol, 1,2-butylene glycol, 1,2-pentylene glycol, 1,2-hexylene glycol, 1,3-propylene glycol, 1,4-butylene glycol, 1,5-pentylene glycol, and 1,6-hexylene glycol. Also preferably, the C₃₋₈alkylene glycols are C₃ or C₄ alkylene glycols such as 1,2-propylene glycol, 1,3-propylene glycol, 1,2-butylene glycol, 1,3-butylene glycol, 1,4-butylene glycol, 2,3-butylene glycol, 2-methyl-1,2-propylene glycol, and 2-methyl-1,3-propylene glycol. Most preferably, the C₃₋₈alkylene glycol is 1,2-propylene glycol or 1,3-propylene glycol. Another highly preferable glycol is 1,6-hexylene glycol.

The lower alkanol, when present, may be used in amounts up to 50 % by weight, but usually is present from 2 % to 30 % by weight, preferably 10 % to 20 % by weight, most preferably 16 % by weight of the entire formulation. Lower alkanol is selected from C₁₋₇, preferably C₁₋₄, straight or branched alkanols, such as methanol, ethanol, n-propanol, isopropanol, n-butanol, sec-butanol and t-butanol, more preferably isopropanol or ethanol, most preferably isopropanol.

When both the glycol and alkanol components are present, they may individually be present in amounts below the foregoing minimums provided that the sum of the % fraction of the two totals at least 1.0. The % fraction is defined as the actual % present divided by the minimum % previously stated (10% for the glycol and 2% for the lower alkanol). For example, a solution having 1 % alkanol has a % fraction of 0.5(1 %/2 %) for alkanol. Such a solution would then require at least a % fraction of glycol of 0.5, or at least 5 % glycol component in the solution. Similarly a 1 % glycol containing solution, having a % fraction of 0.1(1 %/10 %), would require at least an alkanol % fraction of 0.9, or 1.8 % alkanol. Simply put, if the glycol is present in x % and the alkanol y %, then the minimums of the ranges would be the solutions for the equation$\frac{\text{y %}}{\text{minimum of alkanol alone}} \text{+} \frac{\text{x %}}{\text{minimum of glycol alone}} \text{≧ 1.0.}$ The tonicity builder is present in an amount which raises the solution tonicity to the equivalent of a 5 % to 20 % sodium chloride solution (w/v %), preferably to a tonicity in the range of 8.5 % to 17.5 % sodium chloride, more preferably 10 % to 15 % sodium chloride, most preferably 12.5 % sodium chloride. The most preferable compound for use as a tonicity builder is sodium chloride, although any compatible (ocularly compatible if ophthalmic device disinfection is the intended usage) inorganic or organic salt which does not interfere with the other components will do.

The overall solution tonicity should preferably be at least equivalent to 7.5 % to 12.5 %, more preferably 10 % aqueous NaCl. The tonicity builder amounts required can be adjusted by those of ordinary skill to have the solution meet these overall more preferable limits. Typical tonicity builders include ophthalmically acceptable alkaline metal or alkaline earth metal halide, phosphate, carbonate, sulfate, etc. The most preferred tonicity builder is sodium chloride.

The surfactant is present in an amount of from 2 to 25 % by weight but is usually present in an amount of 2 % to 15 % by weight, preferably 3 % to 12 % by weight, most preferably 5 % by weight to 10 % by weight of the entire formulation. The surfactant is selected from virtually any ocularly acceptable surfactant including non-ionic, anionic, and amphoteric surfactants, and furthermore, if the ultimate use is not ophthalmic, the components need not be pharmaceutically acceptable. However, it is preferably selected from

### ca) compounds of formula I

(AmSur-O)₃-P=O (I)

wherein the group AmSur is of the formula 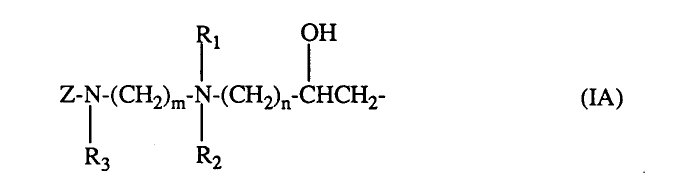
wherein each of R₁ and R₂ is independently lower alkyl, hydroxy lower alkyl, or carboxy lower alkyl, R₃ is hydrogen, lower alkyl, hydroxy lower alkyl, or carboxy lower alkyl, Z is an alkanoyl of 6-18 carbon atoms or Z together with R₁ and R₂ is a carbon substituted by C₅-₁₇alkyl; and n and m are each independently 1 to 4. Where AmSur contains a net charge, a suitable ocularly acceptable counter ion, such as a halogenide, e.g. chloride, is also present in an appropriate amount. The three AmSur radicals can be the same or different, but preferably all three AmSur radicals in one molecule are the same;

### cb) compounds of the formula

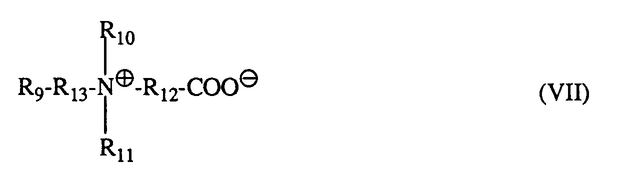

wherein R₉ is alkyl of 5-17 carbon atoms or a C₆₋₂₀alkanoylamino; each of R₁₀ and R₁₁ is independently lower alkyl, hydroxy lower alkyl, or carboxy lower alkyl; R₁₂ is an alpha,omega-alkylene of 1 to 6 carbons which is unsubstituted or substituted by lower alkyl, hydroxy, or hydroxy lower alkyl; and R₁₃ is alpha,omega-C₁₋₅alkylene;

### cc) compounds of the formula

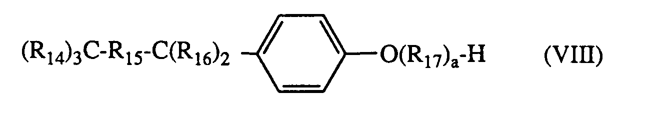

wherein each R₁₄ and each R₁₆ is independently C₁₋₄alkyl; R₁₅ is C₁₋₄-alpha,omega-alkylene; each R₁₇ is independently -CH₂CH₂O-, -CH₂CH₂CH₂O-, or 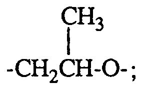
and a is 3-18; and

### cd) compounds of the formula

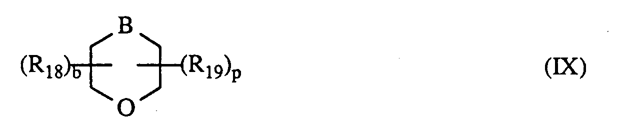

wherein B is a C₁₋₄-alpha,omega-alkylene; p is an integer from 0 to (d-1); b is an integer which is (d-p-1); d is 4 to 7; each R₁₈ is independently H or a C₁₋₄alkyl which is unsubstituted or substituted by at least one R₁₉; each R₁₉ is independently hydroxy which is free, etherified by R₂₀, or esterified by R₂₁; each R₂₀ is a C₂₋₄ straight or branched oxyalkylene or poly(C₂₋₄ straight or branched oxyalkylene), the terminal oxygen of which is bound to H or R₂₁; and each R₂₁ is independently an acyl of a C₂₋₂₄alkanoic acid or a C₄₋₂₄alkenoic acid; provided that in each compound of formula IX there is at least one free hydroxy group, and at least one R₂₁ group. Compounds of formula VII are typically available from Miranol under the names Mirataine® and Miranol®; compounds of formula VIII are available under the names Igepal CA®, Polytergent® and Triton X®; and compounds of formula IX are available under the Span® and Tween® brand names.

Hereinbefore and hereinafter "lower", such as in lower alkyl, refers to residues having up to 7 carbon atoms, preferably up to 4 carbon atoms.

Preferably the compounds of formula I are selected from 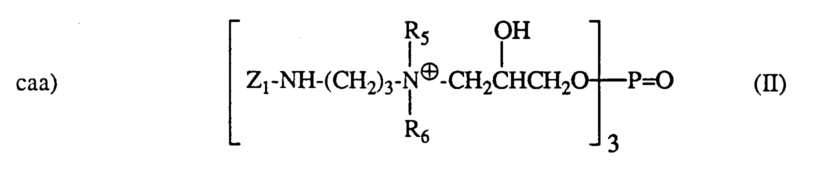
wherein R₅ and R₆ are each C₁₋₄alkyl and Z₁ is C₆₋₁₈alkanoyl; 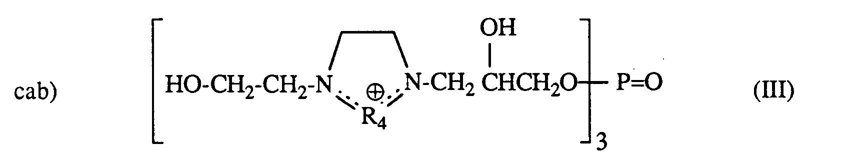
wherein R₄ is a carbon substituted by C₅₋₁₇alkyl and the dotted lines indicate that there is one double bond between R₄ and one of the nitrogens attached to R₄; and 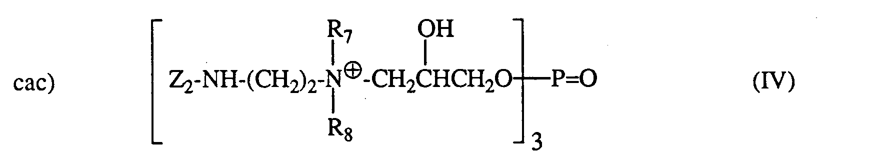
wherein Z₂ is C₁₂₋₁₄alkanoyl, one of R₇ and R₈ is carboxy lower alkyl, and the other of R₇ and R₈ is hydroxy lower alkyl.

Compounds of formulae II-IV are available from Mona Industries, New Jersey under the series trade name Monaquat®-P. More preferably, within formulae II-IV, are the compounds
caaa) [Z₁-NH-(CH₂)₃N^{⊕}(CH₃)₂-CH₂CH(OH)CH₂O]₃-P=O·3Cl^{⊖} (V) wherein Z₁ is C₆₋₁₇-alkanoyl (available under the name Monaquat®P-TC) or C₁₂₋₁₄alkanoyl (available under the name Monaquat®P-TD);
caba) compounds of formula III, available under the name Monaquat®P-TZ; and 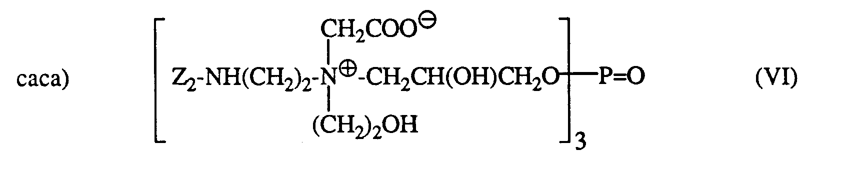
wherein Z₂ is C₁₂₋₁₄alkanoyl, available under the name Monaquat®P-TL. The most preferable compound of the Monaquat®P series for use in the instant invention is Monaquat®P-TL, i.e. compounds of formula VI. Compounds within formula II generally are disclosed in US Patents 4,209,449 and 4,336,385, the disclosures of which are included herein by reference.

Another preferred class of surfactants includes poloxamers, reverse poloxamers, meroxapols, poloxamines, polyethyleneglycols, polypropyleneglycols, polypropyleneglycol-buteths, polypropyleneglycol oleates, polypropylene-pareths, tetrahydroxypropylethylenediamine, ceteareths, NTA salts, EDTA salts, and pentetate salts.

Within this group, especially useful are:
poloxamers 101, 105, 108, 122, 123, 124, 181, 182, 183, 184, 185, 188, 212, 215, 217, 231, 234, 235, 237, 238, 282, 288, 331, 333, 334, 335, 338, 401, 402, 403 and 407;
meroxapols 105, 108, 171, 172, 174, 178, 251, 252, 254, 255, 258, 311, 312, and 314;
poloxamines 304, 504, 701, 702, 704, 707, 901, 904, 908, 1101, 1102, 1104, 1107, 1301, 1302, 1304, 1307, 1501, 1502, 1504, and 1508;
polyethylene glycols selected from PEGs 4, 6, 8, 12, 20, 32, 40, 75, 150, and PEG 6 methyl ether;
polypropylene glycols selected from PPGs 9, 12, 17, 26, and 30;
polypropylene glycol-buteths selected from ppg-5-buteth-7, ppg-7-buteth-10, ppg-12-buteth-16, ppg-20-buteth-30, ppg-28-buteth-35, and ppg-33-buteth-45;
ppg-26-oleate;
ppg-6-pareth;
tetrahydroxypropylethylenediamine;
ceteareth 27 and 55;
trisodium NTA;
trisodium EDTA and tetrasodium EDTA;
EDTA; and
pentasodium pentetate. Each of these compounds can be found in the C.T.F.A. Ingredient Dictionary.

The pH regulating component, when present, can be added as a preformed buffer or can be formed in situ. If the pH of the solution without this component is suitable it is not required, although its presence is desirable. Any ocularly compatible inorganic or organic acid or base or buffer system can be used. Typical buffer systems include the well known phosphate or borate systems. Other suitable organic buffer systems include, without limitation, the lactate, pyruvate, citrate, tartrate, acetate, and laurate systems. Preferably said pH adjusting or regulating agent is selected from i) phosphoric acid, boric acid, lactic acid and citric acid, ii) an ophthalmically acceptable salt thereof, iii) a mixture of said acid and said salt of said acid, iv) an ophthalmically acceptable inorganic acid and v) an ophthalmically acceptable inorganic base.

Most preferably the buffer system used will have a pK in the range of the desired pH range so as to maximize the buffering capacity. The most preferable buffer system is lactic acid/lactate which is preferably formed in situ by the addition of lactic acid alone. In the case of lactic acid/lactate as the pH adjuster (i.e. buffer), the combined lactic acid and lactate are preferably present from about 0.5 to about 2 % by weight of the solution based on lactate ion, more preferably about 0.75 % to about 1.5 %, most preferably about 1.1 % of the solution.

The pH of the final solution may be advantageously in the range of 3 to 7.0, preferably 5 to 7, more preferably about 5.5 to about 6. The lower pHs, while suitable, are advantageous in that minimum disinfecting time is shortened over the same composition at higher pH, but disadvantageous that reestablishment of neutral pH is necessary before a lens is placed back on the eye.

The viscosity enhancer, when present, is present to help increase the solution viscosity to preferably not greater than 100 cps, more preferably not greater than 80 cps, still more preferably not greater than 30 cps, most preferably not greater than 10 cps. Any ocularly compatible non-ionic or quaternary ammonium viscosity enhancer is suitable. Examples of non-ionic viscosity enhancers utilizable on the instant invention include: lower alkyl celluloses (i.e. methyl cellulose, ethyl cellulose, etc.) hydroxy lower alkyl celluloses (i.e. hydroxy methyl cellulose, hydroxy ethyl cellulose, hydroxy propyl cellulose, etc.), hydroxy-lower alkanoyl cellulose, lower alkanoyl cellulose, and carboxy-lower alkyl-cellulose, poloxamers, reverse poloxamers, ethoxylated ethylene diamines, etc.

Preferably, the viscosity enhancer is a cellulose ether, more preferably hydroxy lower alkyl cellulose, most preferably hydroxy ethyl cellulose, such as HECQP 4400 available from Union Carbide. In a most preferred solution, hydroxy ethyl cellulose is the viscosity enhancer and is present in an amount of about 0.1 % by weight of the solution.

Another preferred class of viscosity enhancing agents includes poloxamers, reverse poloxamers, meroxapols, poloxamines, polyethyleneglycols, polypropyleneglycols, polypropyleneglycol-buteths, polypropyleneglycol oleates, polypropylene-pareths, tetrahydroxypropylethylenediamine, ceteareths, NTA salts, EDTA salts, and pentetate salts. Within this group, especially useful are:
poloxamers 101, 105, 108, 122, 123, 124, 181, 182, 183, 184, 185, 188, 212, 215, 217, 231, 234, 235, 237, 238, 282, 288, 331, 333, 334, 335, 338, 401,402, 403, and 407;
meroxapols 105, 108, 171, 172, 174, 178, 251, 252, 254, 255, 258, 311, 312, and 314;
poloxamines 304, 504, 701, 702, 704, 707, 901, 904, 908, 1101, 1102, 1104, 1107, 1301, 1302, 1304, 1307, 1501, 1502, 1504, and 1508;
polyethylene glycols selected from PEGs 4, 6, 8, 12, 20, 32, 40, 75, 150, and PEG 6 methyl ether;
polypropylene glycols selected from PPGs 9, 12, 17, 26, and 30;
polypropylene glycol-buteths selected from ppg-5-buteth-7, ppg-7-buteth-10, ppg-12-buteth-16, ppg-20-buteth-30, ppg-28-buteth-35, and ppg-33-buteth-45;
ppg-26-oleate;
ppg-6-pareth;
tetrahydroxypropylethylenediamine;
ceteareth 27 and 55;
trisodium NTA;
trisodium EDTA and tetrasodium EDTA;
EDTA; and
pentasodium pentetate. Each of these compounds can be found in the C.T.F.A. Ingredient Dictionary.

The solution of the invention can be formulated from the above components in any manner known in the art. For example the solid components can be dissolved directly in the water, either simultaneously or sequentially, with liquid components being added thereto either before or after the solid components. Alternatively the solid components can be triturated with one or more non-water liquid components and this mixture diluted with an appropriate amount of water. It is preferable to dissolve all of the components (other than the viscosity enhancer) first and then mix the viscosity enhancer into this solution. Variations of the above will be apparent to the ordinarily skilled formulator.

The instant solutions are rapid cleaning and disinfecting solutions for a wide range of contact lens and other materials. Typically, one applies a few drops of the solution to the lens material and rubs it for 5 to 30 seconds, preferably 10 to 20 seconds, more preferably about 15 seconds. This is repeated for the opposite surface. The lens is then rinsed in water or normal saline for at least 5 seconds, preferably 10 to 20 seconds, most preferably 15 seconds, and stored in normal saline for at least 20 seconds, preferably 30 seconds to 1.5 minutes, most preferably about 1 minute. Longer storing times are acceptable, but not necessary. The instant solution can be used in the above method for all types of contact lenses; soft lenses, hard lenses, and rigid gas permeable lenses. Such lens materials for which the instant solution can be used include bufilcon A, cabufocon A, crofilcon A, deltafilcon A, deltafilcon B, dimefilcon A, droxifilcon, etafilcon A, hefilcon A, hefilcon B, itafocon A, lidofilcon A, mafilcon A, ocufilcon A, ocufilcon B, optacryl 60, pasifocon A, pasifocon B, pasifocon C, perfilcon A, phemfilcon A, polymacon, porofocon B, silafilcon A, silafocon A, tefilcon, tetrafilcon A, vifilcon A, PMMA, silicone/MMA copolymer, MMA/glyceryl methacrylate copolymer, and poly t-butyl styrene. Others will be apparent to those of ordinary skill.

Having fully described the invention, the following Examples are presented to exemplify the invention.

Example 1: 15.72 g isopropyl alcohol, 10.00 g of sodium chloride, 10.00 g of Pluronic F-127 (poloxamer 407), and 15.00 g of Miranol H2M (concentrate having approximately 50 % solid) are dissolved in 49.3 g of water (deionized) and the pH is adjusted with concentrated HCl to result in a solution according to the invention having a pH of 6.0. Microbiological data is presented in example 32.

Example 2: Example 1 is followed except that the amount of Miranol H2M is 20.00 g and water is 44.3 g. Microbiological data is presented in Table 5.

Examples 3 through 16: Examples 3-16 are prepared in the same manner as example 1 but using the amounts set forth in Table 1 below. In each of these examples, 15.7 g of isopropyl alcohol is employed and the pH is 6.0. Microbiological data is presented in Table 5.

**Table 1**

| Example No. | NaCl (g) | Pluronic F-127 (g) | Miranol H2M (g) | H₂O (g) |
|---|---|---|---|---|
| 3 | 10.0 | 5.0 | 10.0 | 59.3 |
| 4 | 10.0 | 5.0 | 15.0 | 54.3 |
| 5 | 10.0 | 5.0 | 20.0 | 49.3 |
| 6 | 7.0 | 15.0 | 10.0 | 52.3 |
| 7 | 7.0 | 15.0 | 15.0 | 47.3 |
| 8 | 7.0 | 15.0 | 20.0 | 42.3 |
| 9 | 7.0 | 10.0 | 10.0 | 57.3 |
| 10 | 7.0 | 10.0 | 15.0 | 52.3 |
| 11 | 7.0 | 10.0 | 20.0 | 47.3 |
| 12 | 7.0 | 5.0 | 10.0 | 62.3 |
| 13 | 7.0 | 5.0 | 15.0 | 57.3 |
| 14 | 7.0 | 5.0 | 20.0 | 52.3 |
| 15 | 5.0 | 15.0 | 10.0 | 54.3 |
| 16 | 10.0 | 15.0 | 10.0 | 49.3 |

Examples 17-19: Examples 17 to 19 are prepared in accordance with example 1 except that 16 g of isopropyl alcohol are used, the pH is 6.0 and the amounts set forth in Table 2 are employed.

**Table 2**

| Example No. | NaCl (g) | Pluronic F-127 (g) | Miranol H2M (g) | H₂O (g) |
|---|---|---|---|---|
| 17 | 12.0 | 10.0 | 10.0 | 52.0 |
| 18 | 12.0 | 15.0 | 10.0 | 47.3 |
| 19 | 10.0 | 10.0 | 10.0 | 49.3 |

Example 20: Example 20 is the same as example 19 except the amount of isopropyl alcohol is 20 g.

Examples 21-23: Examples 21 to 23 are prepared in accordance with example 1 using hexylene glycol in place of the Miranol H2M. 1 g of Pluronic L-31 and 2 g of lactic acid are used and pH is 3.0 in place of the pluronic, acid, and pH in example 1. The remaining ingredients are used in the amounts shown in Table 3.

**Table 3**

| Example No. | Isopropyl Alcohol (g) | NaCl (g) |
|---|---|---|
| 21 | 20 | 10 |
| 22 | 30 | 10 |
| 23 | 40 | 10 |

Examples 24-31: Examples 24-31 are prepared in accordance with example 1 except that 1 g Betaine, with or without hexylene glycol as stated in Table 3 is used in place of the Miranol H2M and lactic acid (2 g) is used in place of hydrochloric acid and the pH is 3.0. 10 g of Pluronic F 127 is present in each of examples 24-31. The remaining ingredients are set forth in Table 4. The solutions are adjusted to 100 g by the addition of water:

**Table 4**

| Example No. | Hexylene Glycol (g) | Isopropyl Alcohol (g) | NaCl (g) |
|---|---|---|---|
| 24 | 0 | 20 | 10 |
| 25 | 0 | 30 | 10 |
| 26 | 0 | 40 | 10 |
| 27 | 30 | 10 | 10 |
| 28 | 30 | 0 | 12 |
| 29 | 30 | 0 | 10 |
| 30 | 0 | 20 | 12 |
| 31 | 0 | 30 | 12 |

Example 32: The solutions of examples 1 to 16 are tested for their effectiveness against S. epidermidis as follows: The organism is cultured to a density of 10⁸/ml in nutrient broth. 0.01 ml of this inoculum is pipetted onto each side of a vifilcon A (55 % water) soft contact lens and left in contact therewith for 5 minutes. The inoculated lens is then allowed to soak in 2 ml of each of the solutions tested for 0.5 to 1 minute and the number of remaining viable organisms is determined. The results are reported in Table 5 below.

**Table 5**

| Solution of Example | No. of Surviving Viable Organisms (S. epidermidis) | % Reduction in contamination |
|---|---|---|
| 1 | 5.45 x 10² | 99.94 % |
| 2 | 8.65 x 10² | 99.91 % |
| 3 | 0 | 100 % |
| 4 | 50 | 99.995 % |
| 5 | 50 | 99.995 % |
| 6 | 6.6 x 10² | 99.934 % |
| 7 | 1 x 10² | 99.99 % |
| 8 | 6.2 x 10² | 99.94 % |
| 9 | 0 | 100 % |
| 10 | 0 | 100 % |
| 11 | 10 | 99.999 % |
| 12 | 0 | 100 % |
| 13 | 25 | 99.997 % |
| 14 | 1.5 x 10² | 99.98 % |
| 15 | 7.6 x 10² | 99.92 % |
| 16 | 0 | 100 % |

Example 33: Solutions of the examples set forth below are tested for effectiveness against Acanthamoeba castelanii as follows: A 10⁷ cyst pellet is dissolved in 10 ml of test solution to result in a 10⁶ cyst/ml concentration in test solution. At the times specified below, 1 ml is withdrawn and diluted with 49 ml of saline to result in a cyst concentration of 2 x 10⁴ cyst/ml. 0.1 ml of this diluted solution is then added to 10 ml of nutrient media so that the entire nutrient media begins with a 2 x 10³cyst population. The inoculated nutrient media are cultured for 3 weeks at which point effectiveness is assessed as (a) total kill (-) or (b) partial or no kill (+). The results are reported in Table 6 below.

**Table 6**

| Solution Example No. | Exposure Time (min.) | | | | |
|---|---|---|---|---|---|
| | 1 | 2 | 3 | 4 | 5 |
| 9 | + | - | - | - | - |
| 19 | + | - | - | - | - |
| 17 | - | - | - | - | - |
| 21 | + | | | | - |
| 22 | + | | | | - |
| 23 | + | | | | - |
| 24 | + | | | | - |
| 25 | + | | | | - |
| 26 | + | | | | - |
| 27 | - | | | | - |
| 28 | - | | | | - |
| 29 | + | | | | - |
| 30 | + | | | | - |
| 31 | + | | | | - |

## Claims (Claims for the following Contracting State(s): AT, BE, CH, DE, DK, FR, GB, GR, IT, LI, LU, NL, SE)

1. A solution for cleaning and disinfecting contact lenses comprising
a) x % by weight of a C₃₋₈alkylene glycol, and
y % by weight of a C₂₋₆alkanol
wherein x is zero to 50 and y is zero to 30 with the proviso that $\text{x/10 + y/2 ≧1.0}$;
b) an amount of a tonicity builder sufficient to raise the solution tonicity to the equivalent of a 5 to 20 % by weight sodium chloride solution;
c) 2 to 25 % by weight of an ophthalmic device material compatible surfactant;
d) 0 to 2 % by weight of a pH adjusting or regulating agent;
e) 0 % to an amount sufficient to bring the solution viscosity to 100 cps of a viscosity enhancing agent; and
f) the balance of a suitable solvent which is preferably water.

2. The solution of claim 1 comprising
a) 10 to 50 % by weight of a C₃₋₈alkylene glycol and 2 to 30 % by weight of a C₂₋₆alkanol;
b) an amount of an ophthalmically acceptable alkali metal or alkaline earth metal halide, phosphate, carbonate, or sulfate which is sufficient to raise the solution tonicity to a 5 to 20 % by weight sodium chloride solution;
c) 2 to 15 % of an ophthalmic device material compatible surfactant selected from
ca) compounds of formula I
(AmSur-O)₃-P=O (I)
wherein each group AmSur is independently of the formula 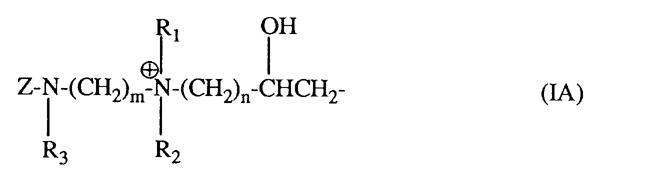 wherein each of R₁ and R₂ is independently lower alkyl, hydroxy lower alkyl, or carboxy lower alkyl, R₃ is hydrogen, lower alkyl, hydroxy lower alkyl, or carboxy lower alkyl, Z is an alkanoyl of 6-18 carbon atoms or Z together with R₁ and R₂ is a carbon substituted by C₅-₁₇alkyl; and n and m are each independently 1 to 4;
cb) compounds of the formula 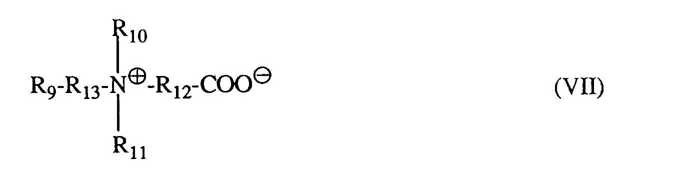 wherein R₉ is alkyl of 5-17 carbon atoms or a C₆₋₂₀alkanoylamino; each of R₁₀ and R₁₁ is independently lower alkyl, hydroxy lower alkyl, or carboxy lower alkyl; R₁₂ is an alpha,omega-alkylene of 1 to 6 carbons which is unsubstituted or substituted by lower alkyl, hydroxy, or hydroxy lower alkyl; and R₁₃ is alpha,omega-C₁₋₅alkylene;
cc) compounds of the formula 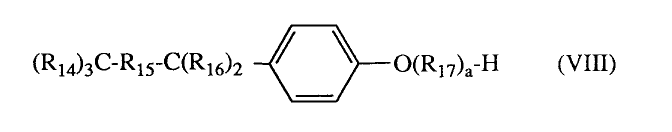 wherein each R₁₄ and each R₁₆ is independently C₁₋₄alkyl; R₁₅ is C₁₋₄-alpha,omega-alkylene; each R₁₇ is independently -CH₂CH₂O-, -CH₂CH₂CH₂O-, or 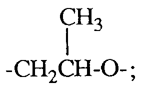 and a is 3-18; and
cd) compounds of the formula 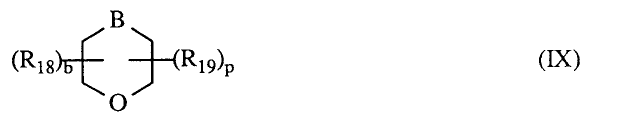 wherein B is a C₁₋₄-alpha,omega-alkylene; p is an integer from 0 to (d-1); b is an integer which is (d-p-1); d is 4 to 7; each R₁₈ is independently H or a C₁₋₄alkyl which is unsubstituted or substituted by at least one R₁₉; each R₁₉ is independently hydroxy which is free, etherified by R₂₀, or esterified by R₂₁; each R₂₀ is a C₂₋₄ straight or branched oxyalkylene or poly(C₂₋₄ straight or branched oxyalkylene), the terminal oxygen of which is bound to H or R₂₁; and each R₂₁ is independently an acyl of a C₂₋₂₄alkanoic acid or a C₄₋₂₄alkenoic acid; provided that in each compound of formula IX there is at least one free hydroxy group, and at least one R₂₁ group;
or poloxamers, reverse poloxamers, meroxapols, poloxamines, polyethyleneglycols, polypropyleneglycols, polypropyleneglycol-buteths, polypropyleneglycol oleates, polypropylene-pareths, tetrahydroxypropylethylenediamine, ceteareths, NTA salts, EDTA salts, and pentetate salts;
d) 0 to 2 % by weight of a pH adjusting or regulating agent selected from i) phosphoric acid, boric acid, lactic acid and citric acid, ii) an ophthalmically acceptable salt thereof, iii) a mixture of said acid and said salt of said acid, iv) an ophthalmically acceptable inorganic acid and v) an ophthalmically acceptable inorganic base;
e) 0 % to an amount sufficient to bring the solution viscosity to 100 cps of a viscosity enhancing agent selected from hydroxy-lower alkyl-cellulose, hydroxy-lower alkanoyl cellulose, lower alkyl-cellulose, lower alkanoyl cellulose, carboxy-lower alkyl-cellulose, poloxamers, reverse poloxamers, meroxapols, poloxamines, polyethyleneglycols, polypropyleneglycols, polypropyleneglycol-buteths, polypropyleneglycol oleates, polypropylene-pareths, tetrahydroxypropylethylenediamine, ceteareths, NTA salts, EDTA salts, and pentetate salts; and
f) water;
wherein the term "lower" refers to residues having up to 7 carbon atoms.

3. The solution of claim 2 wherein the alkylene glycol is omitted.

4. The solution of claim 1 wherein said C₃₋₈alkylene glycol is propylene glycol or hexylene glycol; said C₂₋₆alkanol is ethanol or isopropanol; said tonicity builder is an ophthalmically acceptable alkali metal or alkaline earth metal halide, phosphate, carbonate, or sulfate;
said surfactant is of the formula 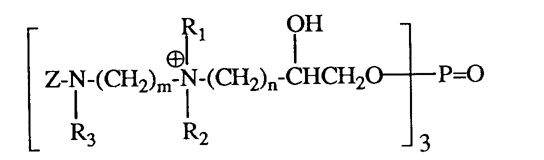
wherein each of R₁ and R₂ is independently lower alkyl, hydroxy lower alkyl, or carboxy lower alkyl, R₃ is hydrogen, lower alkyl, hydroxy lower alkyl, or carboxy lower alkyl, Z is C₆₋₁₈alkanoyl or Z, together with R₁ and R₂, is a carbon substituted by C₅₋₁₇alkyl, and n and m are each 1 to 4, in association with sufficient ions of counter charge to result in a net compound charge of zero;
said pH adjusting or regulating agent is selected from i) phosphoric acid, boric acid, lactic acid and citric acid, ii) an ophthalmically acceptable salt thereof, iii) a mixture of said acid and said salt of said acid, iv) an ophthalmically acceptable inorganic acid and v) an ophthalmically acceptable inorganic base;
said viscosity enhancer is selected from hydroxy-lower alkyl-cellulose, hydroxy-lower alkanoyl cellulose, lower alkyl-cellulose, lower alkanoyl cellulose, and carboxy-lower alkyl-cellulose.

5. The solution of claim 2 wherein said C₃₋₈alkylene glycol is propylene glycol or hexylene glycol; said C₂₋₆alkanol is ethanol or isopropanol; said tonicity builder is sodium chloride;
said surfactant is of the formula 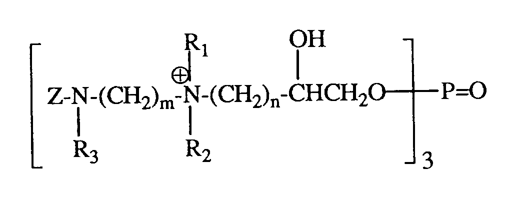 wherein each of R₁ and R₂ is independently lower alkyl, hydroxy lower alkyl, or carboxy lower alkyl, R₃ is hydrogen, lower alkyl, hydroxy lower alkyl, or carboxy lower alkyl, Z is C₆₋₁₈alkanoyl or Z, together with R₁ and R₂, is a carbon substituted by C₅₋₁₇alkyl, and n and m are each 1 to 4, in association with sufficient ions of counter charge to result in a net compound charge of zero; said pH adjusting agent is selected from hydrochloric or lactic acid; and said viscosity enhancer is selected from poloxamer 407 and poloxamer 101.

6. The solution of claim 3 wherein said C₂₋₆alkanol is ethanol or isopropanol; said tonicity builder is sodium chloride;
said surfactant is of the formula 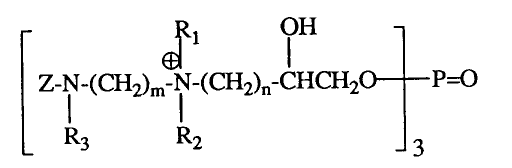 wherein each of R₁ and R₂ is independently lower alkyl, hydroxy lower alkyl, or carboxy lower alkyl, R₃ is hydrogen, lower alkyl, hydroxy lower alkyl, or carboxy lower alkyl, Z is C₆₋₁₈alkanoyl or Z, together with R₁ and R₂, is a carbon substituted by C₅₋₁₇alkyl, and n and m are each 1 to 4, in association with sufficient ions of counter charge to result in a net compound charge of zero; said pH adjusting agent is selected from hydrochloric or lactic acid; and said viscosity enhancer is selected from poloxamer 407 and poloxamer 101.

7. The solution of claim 5 wherein said surfactant is of the formula

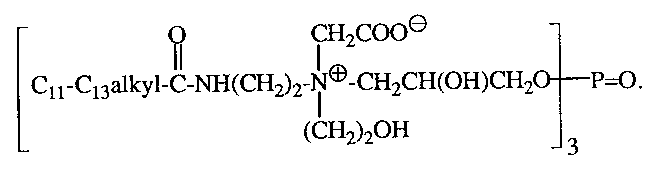

8. The solution of claim 1 wherein said C₃₋₈alkylene glycol is propylene glycol; said lower alkanol is isopropyl alcohol; said tonicity builder is sodium chloride; said surfactant is of the formula 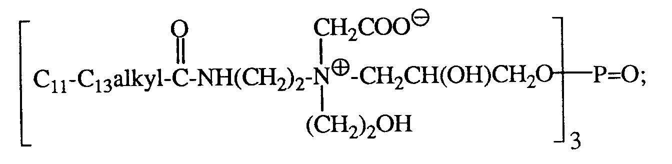 said pH adjusting or regulating agent is lactic acid; and said viscosity enhancer is hydroxy ethyl cellulose.

9. The solution of claim 1 wherein said C₃₋₈alkylene glycol is present in an amount of 21 % by weight; said lower alkanol is present in an amount of 16 % by weight; said surfactant is present in an amount of 5 % by weight; said pH adjusting or regulating agent is present in an amount of 1.1 % by weight; said tonicity builder is present in an amount equivalent to a 12.5 % sodium chloride solution; and said viscosity enhancer is present in an amount of 0.1 % by weight.

10. The solution of claim 1 comprising 0 to 30 % hexylene glycol, 15 to 20 % isopropanol, 7 to 12 % sodium chloride, 5 to 10 % of Miranol, 5 to 15 % of poloxamer 407 or 101 and water.

11. A method of disinfecting and cleaning a contact lens comprising rubbing the surface of said contact lens with an effective disinfecting and cleaning amount of a solution of claim 1 for a period of 5 to 30 seconds, followed by rinsing said contact lens with water or normal saline.

12. The method of claim 11 wherein each surface of said contact lens is rubbed with said solution for 15 seconds and then the entire contact lens is rinsed with normal saline for 10 seconds.

13. The method of claim 12 wherein said rinsing step is followed by storing said contact lens in normal saline for 1 minute.

14. Use of a solution according to any of claims 1 to 10 for disinfecting and cleaning a contact lens.

15. Method of manufacture of a solution of any of claims 1 to 10 characterized by convenient mixing of the individual components.

## Claims (Claims for the following Contracting State(s): ES)

1. Method of manufacture of a solution for cleaning and disinfecting contact lenses comprising
a) x % by weight of a C₃₋₈alkylene glycol, and
y % by weight of a C₂₋₆alkanol
wherein x is zero to 50 and y is zero to 30 with the proviso that $\text{x/10 + y/2≧1.0}$;
b) an amount of a tonicity builder sufficient to raise the solution tonicity to the equivalent of a 5 to 20 % by weight sodium chloride solution;
c) 2 to 25 % by weight of an ophthalmic device material compatible surfactant;
d) 0 to 2 % by weight of a pH adjusting or regulating agent;
e) 0 % to an amount sufficient to bring the solution viscosity to 100 cps of a viscosity enhancing agent; and
f) the balance of a suitable solvent which is preferably water,
characterized by convenient mixing of the individual components.

2. The method of claim 1 comprising mixing
a) 10 to 50 % by weight of a C₃₋₈alkylene glycol and 2 to 30 % by weight of a C₂₋₆alkanol;
b) an amount of an ophthalmically acceptable alkali metal or alkaline earth metal halide, phosphate, carbonate, or sulfate which is sufficient to raise the solution tonicity to a 5 to 20 % by weight sodium chloride solution;
c) 2 to 15 % of an ophthalmic device material compatible surfactant selected from
ca) compounds of formula I
(AmSur-O)₃-P=O (I)
wherein each group AmSur is independently of the formula 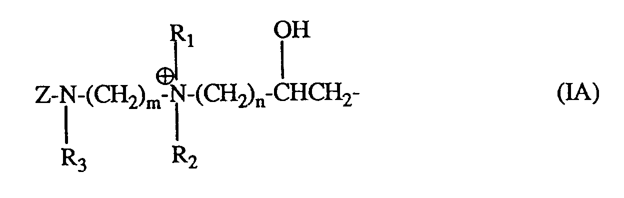 wherein each of R₁ and R₂ is independently lower alkyl, hydroxy lower alkyl, or carboxy lower alkyl, R₃ is hydrogen, lower alkyl, hydroxy lower alkyl, or carboxy lower alkyl, Z is an alkanoyl of 6-18 carbon atoms or Z together with R₁ and R₂ is a carbon substituted by C₅-₁₇alkyl; and n and m are each independently 1 to 4;
cb) compounds of the formula 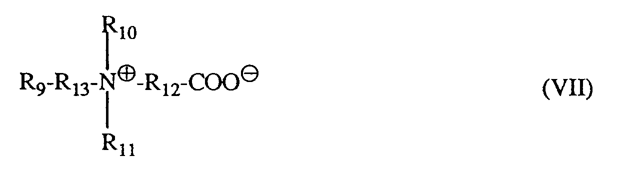 wherein R₉ is alkyl of 5-17 carbon atoms or a C₆₋₂₀alkanoylamino; each of R₁₀ and R₁₁ is independently lower alkyl, hydroxy lower alkyl, or carboxy lower alkyl; R₁₂ is an alpha,omega-alkylene of 1 to 6 carbons which is unsubstituted or substituted by lower alkyl, hydroxy, or hydroxy lower alkyl; and R₁₃ is alpha,omega-C₁₋₅alkylene;
cc) compounds of the formula 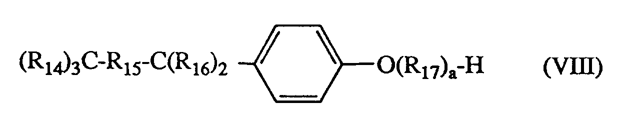 wherein each R₁₄ and each R₁₆ is independently C₁₋₄alkyl; R₁₅ is C₁₋₄-alpha,omega-alkylene; each R₁₇ is independently -CH₂CH₂O-, -CH₂CH₂CH₂O-, or 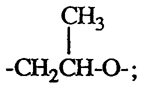 and a is 3-18; and
cd) compounds of the formula 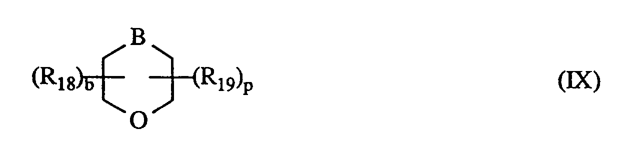 wherein B is a C₁₋₄alpha,omega-alkylene; p is an integer from 0 to (d-1); b is an integer which is (d-p-1); d is 4 to 7; each R₁₈ is independently H or a C₁₋₄alkyl which is unsubstituted or substituted by at least one R₁₉; each R₁₉ is independently hydroxy which is free, etherified by R₂₀, or esterified by R₂₁; each R₂₀ is a C₂₋₄ straight or branched oxyalkylene or poly(C₂₋₄ straight or branched oxyalkylene), the terminal oxygen of which is bound to H or R₂₁; and each R₂₁ is independently an acyl of a C₂₋₂₄alkanoic acid or a C₄₋₂₄alkenoic acid; provided that in each compound of formula IX there is at least one free hydroxy group, and at least one R₂₁ group;
or poloxamers, reverse poloxamers, meroxapols, poloxamines, polyethyleneglycols, polypropyleneglycols, polypropyleneglycol-buteths, polypropyleneglycol oleates, polypropylene-pareths, tetrahydroxypropylethylenediamine, ceteareths, NTA salts, EDTA salts, and pentetate salts;
d) 0 to 2 % by weight of a pH adjusting or regulating agent selected from i) phosphoric acid, boric acid, lactic acid and citric acid, ii) an ophthalmically acceptable salt thereof, iii) a mixture of said acid and said salt of said acid, iv) an ophthalmically acceptable inorganic acid and v) an ophthalmically acceptable inorganic base;
e) 0 % to an amount sufficient to bring the solution viscosity to 100 cps of a viscosity enhancing agent selected from hydroxy-lower alkyl-cellulose, hydroxy-lower alkanoyl cellulose, lower alkyl-cellulose, lower alkanoyl cellulose, carboxy-lower alkyl-cellulose, poloxamers, reverse poloxamers, meroxapols, poloxamines, polyethyleneglycols, polypropyleneglycols, polypropyleneglycol-buteths, polypropyleneglycol oleates, polypropylene-pareths, tetrahydroxypropylethylenediamine, ceteareths, NTA salts, EDTA salts, and pentetate salts; and
f) water;
wherein the term "lower" refers to residues having up to 7 carbon atoms.

3. The method of claim 2 wherein the alkylene glycol is omitted.

4. The method of claim 1 wherein said C₃₋₈alkylene glycol is propylene glycol or hexylene glycol; said C₂₋₆alkanol is ethanol or isopropanol; said tonicity builder is an ophthalmically acceptable alkali metal or alkaline earth metal halide, phosphate, carbonate, or sulfate;
said surfactant is of the formula 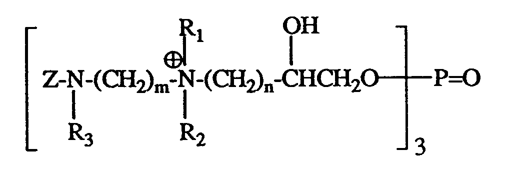
wherein each of R₁ and R₂ is independently lower alkyl, hydroxy lower alkyl, or carboxy lower alkyl, R₃ is hydrogen, lower alkyl, hydroxy lower alkyl, or carboxy lower alkyl, Z is C₆₋₁₈alkanoyl or Z, together with R₁ and R₂, is a carbon substituted by C₅₋₁₇alkyl, and n and m are each 1 to 4, in association with sufficient ions of counter charge to result in a net compound charge of zero;
said pH adjusting or regulating agent is selected from i) phosphoric acid, boric acid, lactic acid and citric acid, ii) an ophthalmically acceptable salt thereof, iii) a mixture of said acid and said salt of said acid, iv) an ophthalmically acceptable inorganic acid and v) an ophthalmically acceptable inorganic base;
said viscosity enhancer is selected from hydroxy-lower alkyl-cellulose, hydroxy-lower alkanoyl cellulose, lower alkyl-cellulose, lower alkanoyl cellulose, and carboxy-lower alkyl-cellulose.

5. The method of claim 2 wherein said C₃₋₈alkylene glycol is propylene glycol or hexylene glycol; said C₂₋₆alkanol is ethanol or isopropanol; said tonicity builder is sodium chloride;
said surfactant is of the formula 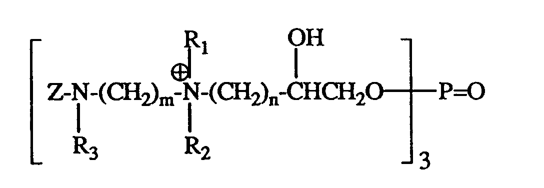 wherein each of R₁ and R₂ is independently lower alkyl, hydroxy lower alkyl, or carboxy lower alkyl, R₃ is hydrogen, lower alkyl, hydroxy lower alkyl, or carboxy lower alkyl, Z is C₆₋₁₈alkanoyl or Z, together with R₁ and R₂, is a carbon substituted by C₅₋₁₇alkyl, and n and m are each 1 to 4, in association with sufficient ions of counter charge to result in a net compound charge of zero; said pH adjusting agent is selected from hydrochloric or lactic acid; and said viscosity enhancer is selected from poloxamer 407 and poloxamer 101.

6. The method of claim 3 wherein said C₂₋₆alkanol is ethanol or isopropanol; said tonicity builder is sodium chloride;
said surfactant is of the formula 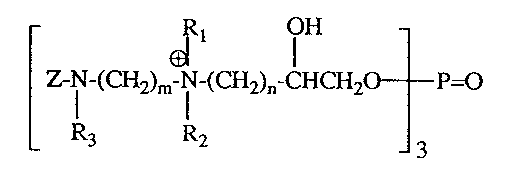 wherein each of R₁ and R₂ is independently lower alkyl, hydroxy lower alkyl, or carboxy lower alkyl, R₃ is hydrogen, lower alkyl, hydroxy lower alkyl, or carboxy lower alkyl, Z is C₆₋₁₈alkanoyl or Z, together with R₁ and R₂, is a carbon substituted by C₅₋₁₇alkyl, and n and m are each 1 to 4, in association with sufficient ions of counter charge to result in a net compound charge of zero; said pH adjusting agent is selected from hydrochloric or lactic acid; and said viscosity enhancer is selected from poloxamer 407 and poloxamer 101.

7. The method of claim 5 wherein said surfactant is of the formula

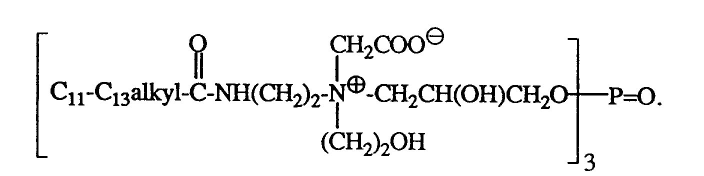

8. The method of claim 1 wherein said C₃₋₈alkylene glycol is propylene glycol; said lower alkanol is isopropyl alcohol; said tonicity builder is sodium chloride; said surfactant is of the formula 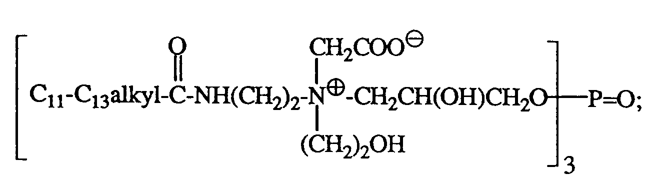 said pH adjusting or regulating agent is lactic acid; and said viscosity enhancer is hydroxy ethyl cellulose.

9. The method of claim 1 wherein said C₃₋₈alkylene glycol is present in an amount of 21 % by weight; said lower alkanol is present in an amount of 16 % by weight; said surfactant is present in an amount of 5 % by weight; said pH adjusting or regulating agent is present in an amount of 1.1 % by weight; said tonicity builder is present in an amount equivalent to a 12.5 % sodium chloride solution; and said viscosity enhancer is present in an amount of 0.1 % by weight.

10. The method of claim 1 comprising mixing 0 to 30 % hexylene glycol, 15 to 20 % isopropanol, 7 to 12% sodium chloride, 5 to 10% of Miranol, 5 to 15 % of poloxamer 407 or 101 and water.

11. A method of disinfecting and cleaning a contact lens comprising rubbing the surface of said contact lens with an effective disinfecting and cleaning amount of a solution of claim 1 for a period of 5 to 30 seconds, followed by rinsing said contact lens with water or normal saline.

12. The method of claim 11 wherein each surface of said contact lens is rubbed with said solution for 15 seconds and then the entire contact lens is rinsed with normal saline for 10 seconds.

13. The method of claim 12 wherein said rinsing step is followed by storing said contact lens in normal saline for 1 minute.

14. Use of a solution according to any of claims 1 to 10 for disinfecting and cleaning a contact lens.

## Patentansprüche (Patentansprüche für folgende(n) Vertragsstaat(en): AT, BE, CH, DE, DK, FR, GB, GR, IT, LI, LU, NL, SE)

1. Lösung zum Reinigen und Desinfizieren von Kontaktlinsen, umfassend
a) x Gewichtsprozent eines C₃₋₈-Alkylenglycols und y Gewichtsprozent eines C₂₋₆-Alkanols, wobei x 0 bis 50 bedeutet und y 0 bis 30 bedeutet, mit der Maßgabe, daß $\text{x/10 + y/2 ≧ 1,0}$;
b) eine Menge eines Tonizitätsbuilders, ausreichend, um die Lösungstonizität auf das Äquivalent einer 5- bis 20-gewichtsprozentigen Natriumchloridlösung anzuheben;
c) 2 bis 25 Gewichtsprozent eines Tensids, das mit einem Material für eine ophthalmische Vorrichtung verträglich ist;
d) 0 bis 2 Gewichtsprozent eines pH einstellenden oder regulierenden Mittels;
e) 0% bis zu einer Menge, die ausreicht, um die Lösungsviskosität auf 100 cP zu bringen, ein viskositätserhöhendes Mittel; und
f) als Rest ein geeignetes Lösungsmittel, das vorzugsweise Wasser ist.

2. Lösung nach Anspruch 1, umfassend
a) 10 bis 50 Gewichtsprozent eines C₃₋₈-Alkylenglycols und 2 bis 30 Gewichtsprozent eines C₂₋₆-Alkanols;
b) eine Menge eines ophthalmisch verträglichen Alkalimetall- oder Erdalkalimetallhalogenids, -phosphats, -carbonats oder -sulfats, ausreichend, um die Lösungstonizität auf die einer 5- bis 20-gewichtsprozentigen Natriumchloridlösung anzuheben;
c) 2 bis 15% eines Tensids, das mit einem Material für eine ophthalmische Vorrichtung verträglich ist, ausgewählt aus
ca) Verbindungen der Formel I
(AmSur-O)₃-P=O (I)
worin jede Gruppe AmSur unabhängig voneinander die Formel 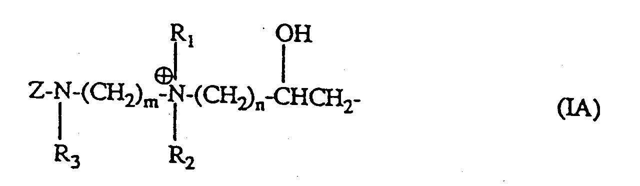 aufweist, worin jeder Rest R₁ und R₂ unabhängig voneinander Niederalkyl, Hydroxyniederalkyl oder Carboxyniederalkyl bedeutet, R₃ Wasserstoff, Niederalkyl, Hydroxyniederalkyl oder Carboxyniederalkyl bedeutet, Z Alkanoyl mit 6-18 Kohlenstoffatomen bedeutet oder Z zusammen mit R₁ und R₂ ein Kohlenstoffatom darstellt, das mit C₅₋₁₇-Alkyl substituiert ist; und n und m jeweils unabhängig voneinander 1 bis 4 bedeuten;
cb) Verbindungen der Formel 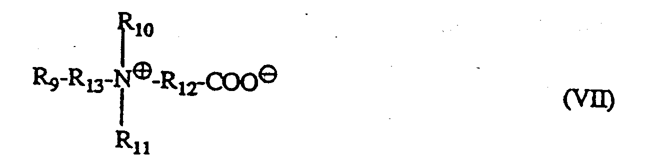 worin R₉ Alkyl mit 5-17 Kohlenstoffatomen oder C₆₋₂₀-Alkanoylamino bedeutet; jeder Rest R₁₀ und R₁₁ unabhängig voneinander Niederalkyl, Hydroxyniederalkyl oder Carboxyniederalkyl bedeutet, R₁₂ ein α,ω-Alkylen mit 1 bis 6 Kohlenstoffatomen bedeutet, das unsubstituiert ist oder mit Niederalkyl, Hydroxy oder Hydroxyniederalkyl substituiert ist und R₁₃ α,ω-C₁₋₅-Alkylen bedeutet;
cc) Verbindungen der Formel 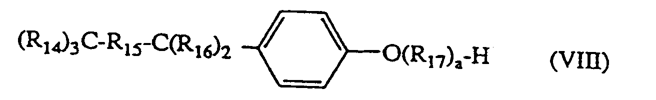 worin jeder Rest R₁₄ und jeder Rest R₁₆ unabhängig voneinander C₁₋₄-Alkyl bedeutet; R₁₅ C₁₋₄-α,ω-Alkylen bedeutet; jeder Rest R₁₇ unabhängig voneinander -CH₂CH₂O-, -CH₂CH₂CH₂O- oder 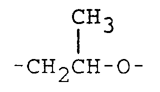 bedeutet und a 3-18 ist; und
cd) Verbindungen der Formel 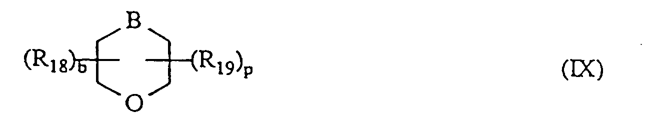 worin B C₁₋₄-α,ω-Alkylen bedeutet; p eine ganze Zahl von 0 bis (d-1) ist; b eine ganze Zahl darstellt, die (d-p-1) ist; d 4 bis 7 ist; jeder Rest R₁₈ unabhängig voneinander H oder C₁₋₄-Alkyl darstellt, unsubstituiert oder substituiert mit mindestens einem, Rest R₁₉; jeder Rest R₁₉ unabhängig voneinander Hydroxy bedeutet, das frei vorliegt oder mit R₂₀ verethert oder mit R₂₁ verestert ist; wobei jeder Rest R₂₀ gerad- oder verzweigtkettiges Oxyalkylen oder Poly(C₂₋₄ gerad- oder verzweigtkettiges Oxyalkylen) darstellt, dessen endständiges Sauerstoffatom an H oder R₂₁ gebunden ist; und jeder Rest R₂₁ unabhängig voneinander ein Acyl einer C₂₋₂₄-Alkansäure oder einer C₄₋₂₄-Alkensäure darstellt; mit der Maßgabe, daß in jeder Verbindung der Formel IX mindestens eine freie Hydroxylgruppe und mindestens eine Gruppe R₂₁ vorliegt, oder Poloxamere, Reverspoloxamere, Meroxapole, Poloxamine, Polyethylenglycole, Polypropylenglycole, Polypropylenglycol-Butethe, Polypropylenglycololeate, Polypropylen-Parethe, Tetrahydroxypropylethylendiamin, Cetearethe, NTA-Salze, EDTA-Salze und Pentetat-Salze;
d) 0 bis 2 Gewichtsprozent eines pH einstellenden oder regulierenden Mittels, ausgewählt aus i) Phosphorsäure, Borsäure, Milchsäure und Zitronensäure, ii) einem ophthalmisch verträglichen Salz davon, iii) einem Gemisch der Säure und des Salzes der Säure, iv) einer ophthalmisch verträglichen anorganischen Säure und v) einer ophthalmisch verträglichen anorganischen Base;
e) 0% bis zu einer Menge, ausreichend, um die Lösungsviskosität auf 100 cP zu bringen, ein viskositätserhöhendes Mittel, ausgewählt aus Hydroxyniederalkylcellulose, Hydroxyniederalkanoylcellulose, Niederalkylcellulose, Niederalkanoylcellulose, Carboxyniederalkylcellulose, Poloxameren, Reverspoloxameren, Meroxapolen, Poloxaminen, Polyethylenglycolen, Polypropylenglycolen, Polypropylenglycol-Butethe , Polypropylenglycololeaten, Polypropylen-Parethen, Tetrahydroxypropylethylendiamin, Cetearethen, NTA-Salzen, EDTA-Salzen und Pentetat-Salzen; und
f) Wasser; wobei der Begriff "nieder" sich auf Reste mit bis zu 7 Kohlenstoffatomen bezieht.

3. Lösung nach Anspruch 2, wobei das Alkylenglycol fortgelassen wird.

4. Lösung nach Anspruch 1, wobei das C₃₋₈-Alkylenglycol Propylenglycol oder Hexylenglycol ist; das C₂₋₆-Alkanol Ethanol oder Isopropanol ist; der Tonizitätsbuilder ein ophthalmisch verträgliches Alkalimetall- oder Erdalkalimetallhalogenid, -phosphat, -carbonat oder -sulfat ist; das Tensid die Formel aufweist 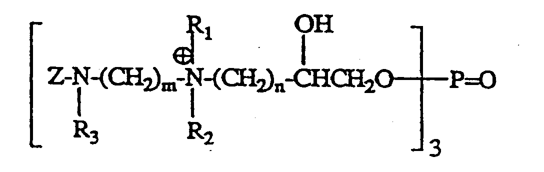 worin jeder der Reste R₁ und R₂ unabhängig voneinander Niederalkyl, Hydroxyniederalkyl oder Carboxyniederalkyl bedeutet, R₃ Wasserstoff, Niederalkyl, Hydroxyniederalkyl oder Carboxyniederalkyl bedeutet, Z C₆₋₁₈-Alkanoyl darstellt oder Z zusammen mit R₁ und R₂ ein Kohlenstoffatom bedeutet, substituiert mit C₅₋₁₇-Alkyl, und n und m jeweils 1 bis 4 sind, in Assoziation mit genügend Gegenladungsionen, die zu einer Nettoladung der Verbindung von 0 führen;
wobei das pH einstellende oder regulierende Mittel ausgewählt ist aus i) Phosphorsäure, Borsäure, Milchsäure und Zitronensäure, ii) einem ophthalmisch verträglichen Salz davon, iii) einem Gemisch der Säure und des Salzes der Säure, iv) einer ophthalmisch verträglichen anorganischen Säure und v) einer ophthalmisch verträglichen anorganischen Base;
wobei das viskositätserhöhende Mittel ausgewählt ist aus Hydroxyniederalkylcellulose, Hydroxyniederalkanoylcellulose, Niederalkylcellulose, Niederalkanoylcellulose und Carboxyniederalkylcellulose.

5. Lösung nach Anspruch 2, wobei das C₃₋₈-Alkylenglycol Propylenglycol oder Hexylenglycol ist; das C₂₋₆-Alkanol Ethanol oder Isopropanol ist; der Tonizitätsbuilder Natriumchlorid ist;
das Tensid die Formel 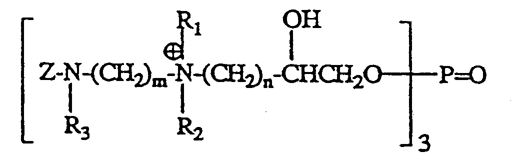 aufweist, worin jeder der Reste R₁ und R₂ unabhängig voneinander Niederalkyl, Hydroxyniederalkyl oder Carboxyniederalkyl bedeutet, R₃ Wasserstoff, Niederalkyl, Hydroxyniederalkyl oder Carboxyniederalkyl bedeutet, Z C₆₋₁₈-Alkanoyl darstellt oder Z zusammen mit R₁ und R₂ ein Kohlenstoffatom bedeutet, substituiert mit C₅₋₁₇-Alkyl, und n und m jeweils 1 bis 4 sind, in Assoziation mit genügend Gegenladungsionen, die zu einer Nettoladung der Verbindung von 0 führen; wobei das pH einstellende Mittel aus Salz- oder Milchsäure ausgewählt ist; und das viskositätserhöhende Mittel aus Poloxamer 407 und Poloxamer 101 ausgewählt ist.

6. Lösung nach Anspruch 3, wobei das C₂₋₆-Alkanol Ethanol oder Isopropanol ist und der Tonizitätsbuilder Natriumchlorid ist; das Tensid die Formel aufweist 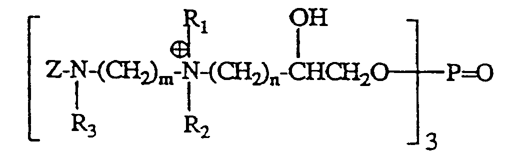 worin jeder Rest R₁ und R₂ unabhängig voneinander Niederalkyl, Hydroxyniederalkyl oder Carboxyniederalkyl bedeutet, R₃ Wasserstoff, Niederalkyl, Hydroxyniederalkyl oder Carboxyniederalkyl bedeutet, Z C₆₋₁₈-Alkanoyl darstellt oder Z zusammen mit R₁ und R₂ ein Kohlenstoffatom bedeutet, substituiert mit C₅₋₁₇-Alkyl, und n und m jeweils 1 bis 4 sind, in Assoziation mit genügend Gegenladungsionen, die zu einer Nettoladung der Verbindung von 0 führen; wobei das pH einstellende Mittel aus Salz- oder Milchsäure ausgewählt ist; und das viskositätserhöhende Mittel aus Poloxamer 407 und Poloxamer 101 ausgewählt ist.

7. Lösung nach Anspruch 5, wobei das Tensid die Formel 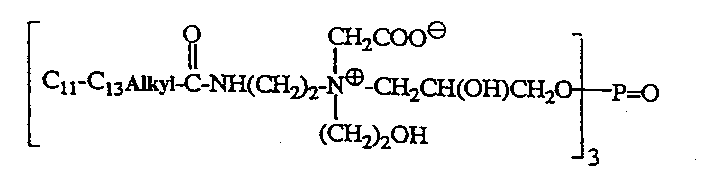 aufweist.

8. Lösung nach Anspruch 1, wobei das C₃₋₈-Alkylenglycol Propylenglycol ist; das Niederalkanol Isopropylalkohol ist; der Tonizitätsbuilder Natriumchlorid ist; und das Tensid die Formel aufweist 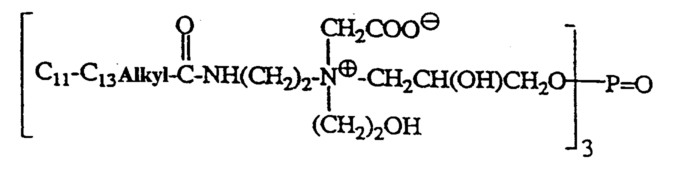 wobei das pH einstellende oder regulierende Mittel Milchsäure ist und das viskositätserhöhende Mittel Hydroxyethylcellulose ist.

9. Lösung nach Anspruch 1, wobei das C₃₋₈-Alkylenglycol in einer Menge von 21 Gewichtsprozent vorliegt; das Niederalkanol in einer Menge von 16 Gewichtsprozent vorliegt; das Tensid in einer Menge von 5 Gewichtsprozent vorliegt; das pH einstellende oder regulierende Mittel in einer Menge von 1,1 Gewichtsprozent vorliegt; der Tonizitätsbuilder in einer Menge äquivalent einer 12,5 gewichtsprozentigen Natriumchloridlösung vorliegt; und das viskositätserhöhende Mittel in einer Menge von 0,1 Gewichtsprozent vorliegt.

10. Lösung nach Anspruch 1, umfassend 0 bis 30% Hexylenglycol, 15 bis 20% Isopropanol, 7 bis 12% Natriumchlorid, 5 bis 10% Miranol, 5 bis 15% Poloxamer 407 oder 101 und Wasser.

11. Verfahren zur Desinfektion und Reinigung einer Kontaktlinse, umfassend Reiben der Oberfläche der Kontaktlinse mit einer wirksam desinfizierenden und reinigenden Menge einer Lösung nach Anspruch 1 für einen Zeitraum von 5 bis 30 Sekunden, gefolgt von Spülen der Kontaktlinse mit Wasser oder normaler Salzlösung.

12. Verfahren nach Anspruch 11, wobei jede Oberfläche der Kontaktlinse mit der Lösung für 15 Sekunden gerieben wird und anschließend die gesamte Kontaktlinse mit normaler Salzlösung für 10 Sekunden gespült wird.

13. Verfahren nach Anspruch 12, wobei dem Spülschritt Lagern der Kontaktlinse in normaler Salzlösung für 1 Minute folgt.

14. Verwendung einer Lösung nach einem der Ansprüche 1 bis 10 zur Desinfektion und Reinigung einer Kontaktlinse.

15. Verfahren zur Herstellung einer Lösung nach einem der Ansprüche 1 bis 10, dadurch gekennzeichnet, daß die einzelnen Komponenten in geeigneter Weise vermischt werden.

## Patentansprüche (Patentansprüche für folgende(n) Vertragsstaat(en): ES)

1. Verfahren zur Herstellung einer Lösung zum Reinigen und Desinfizieren von Kontaktlinsen, umfassend
a) x Gewichtsprozent eines C₃₋₈-Alkylenglycols und y Gewichtsprozent eines C₂₋₆-Alkanols, wobei x 0 bis 50 bedeutet und y 0 bis 30 bedeutet, mit der Maßgabe, daß $\text{x/10 + y/2 ≧ 1,0}$;
b) eine Menge eines Tonizitätsbuilders, ausreichend, um die Lösungstonizität auf das Äquivalent einer 5- bis 20-gewichtsprozentigen Natriumchloridlösung anzuheben;
c) 2 bis 25 Gewichtsprozent eines Tensids, das mit einem Material für eine ophthalmische Vorrichtung verträglich ist;
d) 0 bis 2 Gewichtsprozent eines pH einstellenden oder regulierenden Mittels;
e) 0% bis zu einer Menge, die ausreicht, um die Lösungsviskosität auf 100 cP zu bringen, ein viskositätserhöhendes Mittel; und
f) als Rest ein geeignetes Lösungsmittel, das vorzugsweise Wasser ist, gekennzeichnet durch geeignetes Vermischen der einzelnen Komponenten.

2. Verfahren nach Anspruch 1, umfassend Vermischen von
a) 10 bis 50 Gewichtsprozent eines C₃₋₈-Alkylenglycols und 2 bis 30 Gewichtsprozent eines C₂₋₆-Alkanols;
b) einer Menge eines ophthalmisch verträglichen Alkalimetall- oder Erdalkalimetallhalogenids, -phosphats, -carbonats oder -sulfats, ausreichend, um die Lösungstonizität auf die einer 5- bis 20-gewichtsprozentigen Natriumchloridlösung anzuheben;
c) 2 bis 15% eines Tensids, das mit einem Material für eine ophthalmische Vorrichtung verträglich ist, ausgewählt aus
ca) Verbindungen der Formel I
(AmSur-O)₃-P=O (I)
worin jede Gruppe AmSur unabhängig voneinander die Formel 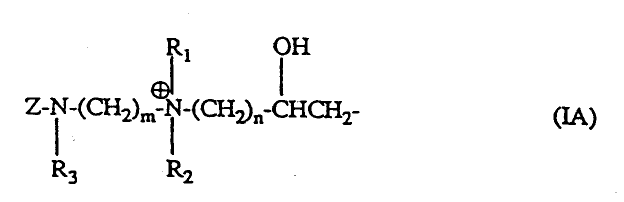 aufweist, ,worin jeder Rest R₁ und R₂ unabhängig voneinander Niederalkyl, Hydroxyniederalkyl oder Carboxyniederalkyl bedeutet, R₃ Wasserstoff, Niederalkyl, Hydroxyniederalkyl oder Carboxyniederalkyl bedeutet, Z Alkanoyl mit 6-18 Kohlenstoffatomen bedeutet oder Z zusammen mit R₁ und R₂ ein Kohlenstoffatom darstellt, das mit C₅₋₁₇-Alkyl substituiert ist; und n und m jeweils unabhängig voneinander 1 bis 4 bedeuten;
cb) Verbindungen der Formel 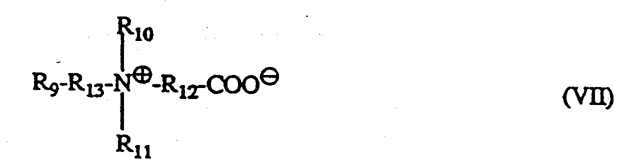 worin R₉ Alkyl mit 5-17 Kohlenstoffatomen oder C₆₋₂₀-Alkanoylamino bedeutet; jeder Rest R₁₀ und R₁₁ unabhängig voneinander Niederalkyl, Hydroxyniederalkyl oder Carboxyniederalkyl bedeutet, R₁₂ ein α,ω-Alkylen mit 1 bis 6 Kohlenstoffatomen bedeutet, das unsubstituiert ist oder mit Niederalkyl, Hydroxy oder Hydroxyniederalkyl substituiert ist und R₁₃ α,ω-C₁₋₅-Alkylen bedeutet;
cc) Verbindungen der Formel 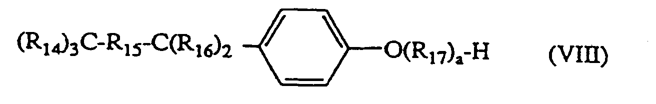 worin jeder Rest R₁₄ und jeder Rest R₁₆ unabhängig voneinander C₁₋₄-Alkyl bedeutet; R₁₅ C₁₋₄-α,ω-Alkylen bedeutet; jeder Rest R₁₇ unabhängig voneinander -CH₂CH₂O-, -CH₂CH₂CH₂O- oder 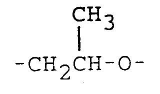 bedeutet und a 3-18 ist; und
cd) Verbindungen der Formel 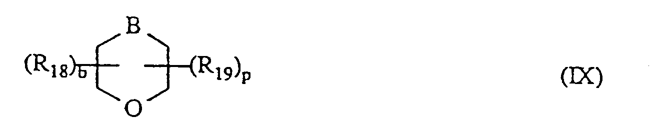 worin B C₁₋₄-α,ω-Alkylen bedeutet; p eine ganze Zahl von 0 bis (d-1) ist; b eine ganze Zahl darstellt, die (d-p-1) ist; d 4 bis 7 ist; jeder Rest R₁₈ unabhängig voneinander H oder C₁₋₄-Alkyl darstellt, unsubstituiert oder substituiert mit mindestens einem Rest R₁₉; jeder Rest R₁₉ unabhängig voneinander, Hydroxy bedeutet, das frei vorliegt oder mit R₂₀ verethert oder mit R₂₁ verestert ist; wobei jeder Rest R₂₀ gerad- oder verzweigtkettiges Oxyalkylen oder Poly(C₂₋₄ gerad- oder verzweigtkettiges Oxyalkylen) darstellt, dessen endständiges Sauerstoffatom an, H oder R₂₁ gebunden ist; und jeder Rest R₂₁ unabhängig voneinander ein Acyl einer C₂₋₂₄-Alkansäure oder einer C₄₋₂₄-Alkensäure darstellt; mit der Maßgabe, daß in jeder Verbindung der Formel IX mindestens eine freie Hydroxylgruppe und mindestens eine Gruppe R₂₁ vorliegt, oder Poloxamere, Reverspoloxamere, Meroxapole, Poloxamine, Polyethylenglycole, Polypropylenglycole, Polypropylenglycol-Butethe, Polypropylenglycololeate, Polypropylen-Parethe, Tetrahydroxypropylethylendiamin, Cetearethe, NTA-Salze, EDTA-Salze und Pentetat-Salze;
d) 0 bis 2 Gewichtsprozent eines pH einstellenden oder regulierenden Mittels, ausgewählt aus i) Phosphorsäure, Borsäure, Milchsäure und Zitronensäure, ii) einem ophthalmisch verträglichen Salz davon, iii) einem Gemisch der Säure und des Salzes der Säure, iv) einer ophthalmisch verträglichen anorganischen Säure und v) einer ophthalmisch verträglichen anorganischen Base;
e) 0% bis zu einer Menge, ausreichend, um die Lösungsviskosität auf 100 cP zu bringen, ein viskositätserhöhendes Mittel, ausgewählt aus Hydroxyniederalkylcellulose, Hydroxyniederalkanoylcellulose, Niederalkylcellulose, Niederalkanoylcellulose, Carboxyniederalkylcellulose, Poloxameren, Reverspoloxameren, Meroxapolen, Poloxaminen, Polyethylenglycolen, Polypropylenglycolen, Polypropylenglycol-Butethen, Polypropylenglycololeaten, Polypropylen-Parethen, Tetrahydroxypropylethylendiamin, Cetearethen, NTA-Salzen, EDTA-Salzen und Pentetat-Salzen; und
f) Wasser; wobei der Begriff "nieder" sich auf Reste mit bis zu 7 Kohlenstoffatomen bezieht.

3. Verfahren nach Anspruch 2, wobei das Alkylenglycol fortgelassen wird.

4. Verfahren nach Anspruch 1, wobei das C₃₋₈-Alkylenglycol Propylenglycol oder Hexylenglycol ist; das C₂₋₆-Alkanol Ethanol oder Isopropanol ist; der Tonizitätsbuilder ein ophthalmisch verträgliches Alkalimetall- oder Erdalkalimetallhalogenid, -phosphat, -carbonat oder -sulfat ist; das Tensid die Formel aufweist 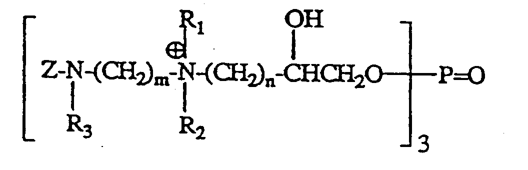 worin jeder der Reste R₁ und R₂ unabhängig voneinander Niederalkyl, Hydroxyniederalkyl oder Carboxyniederalkyl bedeutet, R₃ Wasserstoff, Niederalkyl, Hydroxyniederalkyl oder Carboxyniederalkyl bedeutet, Z C₆₋₁₈-Alkanoyl darstellt oder Z zusammen mit R₁ und R₂ ein Kohlenstoffatom bedeutet, substituiert mit C₅₋₁₇-Alkyl, und n und m jeweils 1 bis 4 sind, in Assoziation mit genügend Gegenladungsionen, die zu einer Nettoladung der Verbindung von 0 führen;
wobei das pH einstellende oder regulierende Mittel ausgewählt ist aus i) Phosphorsäure, Borsäure, Milchsäure und Zitronensäure, ii) einem ophthalmisch verträglichen Salz davon, iii) einem Gemisch der Säure und des Salzes der Säure, iv) einer ophthalmisch verträglichen anorganischen Säure und v) einer ophthalmisch verträglichen anorganischen Base;
wobei das viskositätserhöhende Mittel ausgewählt ist aus Hydroxyniederalkylcellulose, Hydroxyniederalkanoylcellulose, Niederalkylcellulose, Niederalkanoylcellulose und Carboxyniederalkylcellulose.

5. Verfahren nach Anspruch 2, wobei das C₃₋₈-Alkylenglycol Propylenglycol oder Hexylenglycol ist; das C₂₋₆-Alkanol Ethanol oder Isopropanol ist; der Tonizitätsbuilder Natriumchlorid ist;
das Tensid die Formel 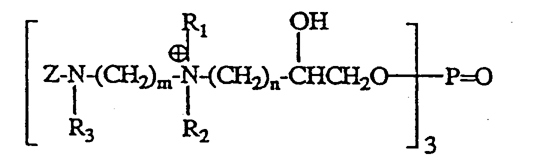 aufweist, worin jeder der Reste R₁ und R₂ unabhängig voneinander Niederalkyl, Hydroxyniederalkyl oder Carboxyniederalkyl bedeutet, R₃ Wasserstoff, Niederalkyl, Hydroxyniederalkyl oder Carboxyniederalkyl bedeutet, Z C₆₋₁₈-Alkanoyl darstellt oder Z zusammen mit R₁ und R₂ ein Kohlenstoffatom bedeutet, substituiert mit C₅₋₁₇-Alkyl, und n und m jeweils 1 bis 4 sind, in Assoziation mit genügend Gegenladungsionen, die zu einer Nettoladung der Verbindung von 0 fuhren; wobei das pH einstellende Mittel aus Salz- oder Milchsäure ausgewählt ist; und das viskositätserhöhende Mittel aus Poloxamer 407 und Poloxamer 101 ausgewählt ist.

6. Verfahren nach Anspruch 3, wobei das C₂₋₆-Alkanol Ethanol oder Isopropanol ist und der Tonizitätsbuilder Natriumchlorid ist; das Tensid die Formel aufweist 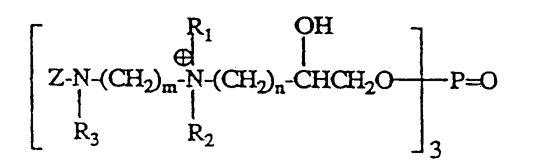 worin jeder Rest R₁ und R₂ unabhängig voneinander Niederalkyl, Hydroxyniederalkyl oder Carboxyniederalkyl bedeutet, R₃ Wasserstoff, Niederalkyl, Hydroxyniederalkyl oder Carboxyniederalkyl bedeutet, Z C₆₋₁₈-Alkanoyl darstellt oder Z zusammen mit R₁ und R₂ ein Kohlenstoffatom bedeutet, substituiert mit C₅₋₁₇-Alkyl, und n und m jeweils 1 bis 4 sind in Assoziation mit genügend Gegenladungsionen, die zu einer Nettoladung der Verbindung von 0 führen; wobei das pH einstellende Mittel aus Salz- oder Milchsäure ausgewählt ist; und das viskositätserhöhende Mittel aus Poloxamer 407 und Poloxamer 101 ausgewählt ist.

7. Verfahren nach Anspruch 5, wobei das Tensid die Formel 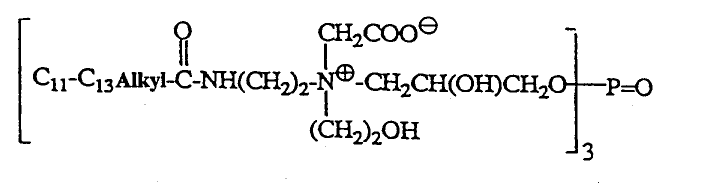 aufweist.

8. Verfahren nach Anspruch 1, wobei das C₃₋₈-Alkylenglycol Propylenglycol ist; das Niederalkanol Isopropylalkohol ist; der Tonizitätsbuilder Natriumchlorid ist; und das Tensid die Formel aufweist 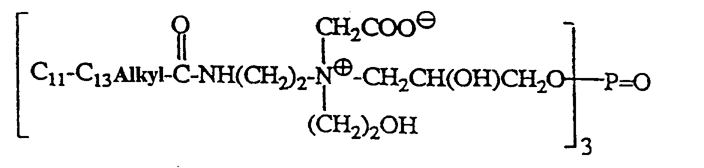 wobei das pH einstellende oder regulierende Mittel Milchsäure ist und das viskositätserhöhende Mittel Hydroxyethylcellulose ist.

9. Verfahren nach Anspruch 1, wobei das C₃₋₈-Alkylenglycol in einer Menge von 21 Gewichtsprozent vorliegt; das Niederalkanol in einer Menge von 16 Gewichtsprozent vorliegt; das Tensid in einer Menge von 5 Gewichtsprozent vorliegt; das pH einstellende oder regulierende Mittel in einer Menge von 1,1 Gewichtsprozent vorliegt; der Tonizitätsbuilder in einer Menge äquivalent einer 12,5 gewichtsprozentigen Natriumchloridlösung vorliegt; und das viskositätserhöhende Mittel in einer Menge von 0,1 Gewichtsprozent vorliegt.

10. Verfahren nach Anspruch 1, umfassend Vermischen von 0 bis 30% Hexylenglycol, 15 bis 20% Isopropanol, 7 bis 12% Natriumchlorid, 5 bis 10% Miranol, 5 bis 15% Poloxamer 407 oder 101 und Wasser.

11. Verfahren zur Desinfektion und Reinigung einer Kontaktlinse, umfassend Reiben der Oberfläche der Kontaktlinse mit einer wirksam desinfizierenden und reinigenden Menge einer Lösung nach Anspruch 1 für einen Zeitraum von 5 bis 30 Sekunden, gefolgt von Spülen der Kontaktlinse mit Wasser oder normaler Salzlösung.

12. Verfahren nach Anspruch 11, wobei jede Oberfläche der Kontaktlinse mit der Lösung für 15 Sekunden gerieben wird und anschließend die gesamte Kontaktlinse mit normaler Salzlösung für 10 Sekunden gespült wird.

13. Verfahren nach Anspruch 12, wobei dem Spülschritt Lagern der Kontaktlinse in normaler Salzlösung für 1 Minute folgt.

14. Verwendung einer Lösung nach einem der Ansprüche 1 bis 10 zur Desinfektion und Reinigung einer Kontaktlinse.

## Revendications (Revendications pour l'(les) Etat(s) contractant(s) suivant(s): AT, BE, CH, DE, DK, FR, GB, GR, IT, LI, LU, NL, SE)

1. Une solution pour nettoyage et désinfection des lentilles de contact, qui comporte:
a) x % en poids d'un alkylène (en C₃ à C₈) glycol et
y % en poids d'un alcanol en C₂ à C₆,
x étant de zéro à 50 tandis que y est de zéro à 30 sous la condition que $\text{x/10 + y/2 ≧ 1,0}$;
b) une quantité d'une charge de tonicité, suffisante pour amener la tonicité de la solution à l'équivalent d'une solution de chlorure de sodium de 5 à 20%;
c) de 2 à 25% en poids d'un agent tensioactif compatible avec le matériau constitutif du dispositif ophtalmique;
d) de 0 à 2% en poids d'un agent d'ajustement ou de régulation du pH;
e) de 0 % à une quantité suffisante pour amener la viscosité de la solution à 100 cps d'un agent-améliorant la viscosité; et
f) le reste étant un solvant approprié, qui est de préférence de l'eau.

2. La solution de la revendication, comportant:
a) de 10 à 50% en poids d'un alkylène (en C₃ à C₈) glycol et de 2 à 30% en poids d'un alcanol en C₂ à C₆.
b) une quantité d'un halogénure, d'un phosphate, d'un carbonate ou d'un sulfate de métal alcalin ou de métal alcalino terreux, acceptable du point de vue ophtalmique qui est suffisante pour amener la tonicité de la solution à celle d'une solution de 5 à 20% en poids de chlorure de sodium;
c) de 2 à 15% d'un agent tensioactif compatible avec un matériau constitutif d'un dispositif ophtalmique, choisi parmi:
ca) les composés de formule I
(AmSur-O)₃-P=O (I)
dans laquelle le groupe AmSur correspond à la formule 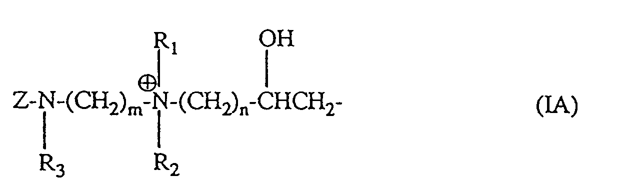 dans laquelle chacun des R₁ et R₂ représente, de façon indépendante, un radical alkyle inférieur, hydroxy-alkyle inférieur ou carboxy-alkyle inférieur, R₃ est de l'hydrogène, un groupe alkyle inférieur, hydroxy-alkyle inférieur ou carboxy-alkyle inférieur, Z est un alcanoyle de 6 à 18 atomes de carbone ou bien Z conjointement avec R₁ et R₂ représente un carbone substitué par un alkyle en C₅ à C₁₇; et n et m représentent chacun de façon indépendante, de 1 à 4;
cb) composés de la formule 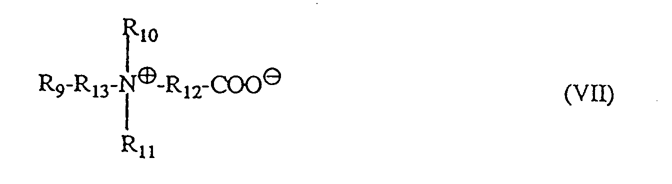 dans laquelle R₉ est un alkyle de 5 à 17 atomes de carbone ou un alcanoyl (en C₆ à C₂₀) amino; chacun des R₁₀ et R₁₁ représentent indépendamment un radical alkyle inférieur, hydroxyl-alkyle inférieur ou carboxy-alkyle inférieur; R₁₂ est un alpha,oméga-alkylène de 1 à 6 atomes de carbone qui est non-substitué ou substitué par un groupe alkyle inférieur, hydroxy ou hydroxy-alkyle inférieur et R₁₃ est un alpha, oméga-alkylène en C₁ à C₅;
cc) composés de la formule 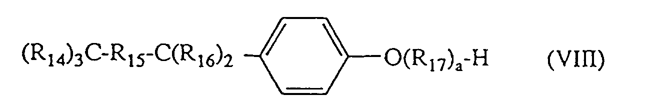 dans laquelle chaque R₁₄ et chaque R₁₆ représentent indépendamment un alkyle en C₁ à C₄; R₁₅ est un alpha,oméga-alkyle en C₁ à C₄; chaque R₁₇ représente indépendamment -CH₂CH₂O-, -CH₂CH₂CH₂O-, ou -CH₂CH(CH₃)-O-et a est de 3 à 18; et
cd) composés de la formule 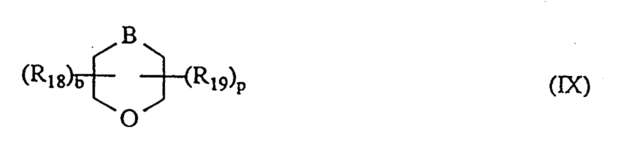 dans laquelle B est un alpha,oméga-alkylène en C₁ à C₄; p est un nombre entier de 0 à (d-1); b est un nombre entier qui est (d-p-1); d est de 4 à 7; chaque R₁₈ représente indépendamment H ou un alkyle en C₁ à C₄ qui est non-substitué ou substitué par au moins un R₁₉; chaque R₁₉ représente indépendamment un radical hydroxy qui est libre, éthérifié par R₂₀ ou bien estérifié par R₂₁; chaque R₂₀ est un oxyalkylène à chaîne droite ou ramifiée en C₂ à C₄ ou un poly(oxyalkylène à chaîne droite ou ramifiée en C₁ à C₄), dont l'oxygène terminal est lié à H ou R₂₁; et chaque R₂₁ représente indépendamment un acyle d'un acide alcanoïque en C₂ à C₂₄ ou d'un acide alcénoïque en C₄ à C₂₄, sous la condition que dans chaque groupe de formule IX, il existe au moins un groupe hydroxyle libre et au moins un groupe R₂₁;
ou bien les poloxyamères, les poloxyamères inversés, les méroxapols, les poloxamines, les polyéthylèneglycols, les polypropylèneglycols, les propylèneglycol-buteths, les oléates de polypropylèneglycol, les polypropylène-pareths, la tétrahydroxypropyl-éthylènediamine, les cétéareths, les sels NTA, les sels d'EDTA et les sels de pentétate;
d) de 0 à 2% en poids d'un agent d'ajustement ou de réglage du pH choisi parmi i) l'acide phosphorique, l'acide borique, l'acide lactique et l'acide citrique, ii) un sel acceptable du point de vue ophtalmique de ceux-ci, iii) un mélange dudit acide et d'un sel dudit acide, iv) un acide inorganique acceptable du point de vue ophtalmique et v) d' une base inorganique acceptable du point de vue ophtalmique;
e) de 0 % à une quantité suffisante pour amener la viscosité de la solution à 100 cps d'un agent améliorant la viscosité choisi parmi les hydroxy-alkyl inférieur-celluloses, les hydroxy alcanoyl inférieur-celluloses, les alkyl inférieur-celluloses, les alcanoyl inférieur-celluloses, les carboxy-alkyl inférieur-celluloses, les polaxamères, les polyéthylèneglycols, les polypropylèneglycols, les propylèneglycol-buteths, les oléates de polypropylèneglycol, les polypropylène-pareths, la tétrahydroxypropyl-éthylènediamine, les cétéareths, les sels NTA, les sels d'EDTA et les sels de pentétate; et
f) de l'eau;
la dénomination "inférieur" se rapportant aux résidus ayant jusqu'à 7 atomes de carbone.

3. La solution de la revendication 2 dans laquelle l'alkylène glycol est omis.

4. La solution de la revendication 1, dans laquelle ledit alkylène en C₃ à C₈ glycol est du propylène glycol ou de l'hexylène glycol; ledit alcanol en C₂ à C₆ est de l'éthanol ou de l'isopropanol; ladite charge de tonicité est un halogénure, un phosphate, un carbonate ou un sulfate de métal alcalin ou alcalino-terreux, acceptable du point de vue ophtalmique;
ledit agent tensioactif correspond à la formule: 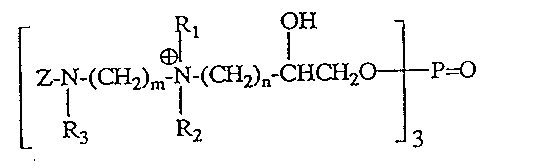 dans laquelle chacune des R₁ et R₂ représente de façon indépendante, un alkyle inférieur, un hydroxy-alkyle inférieur ou un carboxy-alkyle inférieur, R₃ est de l'hydrogène, un alkyle inférieur, un hydroxy-alkyle inférieur ou un carboxy-alkyle inférieur, Z est un alcanoyle en C₆ à C₁₈, ou bien Z, conjointement avec R₁ et R₂, constitue un carbone substitué par un alkyle en C₅ a C₁₇, et n et m représentent chacun de 1 à 4, en association avec suffisamment d'ions de charge contraire pour se traduire par une charge nette du composé de zéro; ledit agent d'ajustement ou de réglage du pH est choisi parmi i) l'acide phosphorique, l'acide borique, l'acide lactique et l'acide citrique, ii) un sel acceptable du point de vue ophtalmique de ceux-ci, iii) un mélange dudit acide et d'un sel dudit acide, iv) un acide inorganique acceptable du point de vue ophtalmique et v) d'une base inorganique acceptable du point de vue ophtalmique;
ledit agent améliorant la viscosité est choisi parmi les hydroxy-alkyl inférieurs-cellulose, les hydroxy-alcanoyl inférieurs-cellulose, les alkyl inférieurs-cellulose, les alcanoyl inférieurs-cellulose et les carboxy-alkyl inférieurs-cellulose.

5. La solution de la revendication 2, dans laquelle l'alkylène glycol en C₃ à C₈ est du propylène glycol ou de l'hexylène glycol, ledit alcanol en C₂ à C₆ est de l'éthanol ou de l'isopropanol; ladite charge de tonicité est du chlorure de sodium;
ledit agent tensioactif correspondant à la formule: 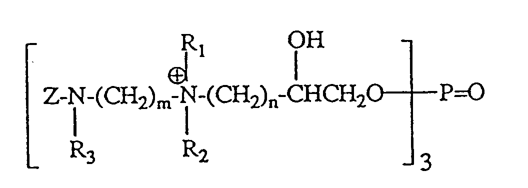 dans laquelle chacune des R₁ et R₂ représente de façon indépendante, un alkyle inférieur, un hydroxy-alkyle inférieur ou un carboxy-alkyle inférieur, R₃ est de l'hydrogène, un alkyle inférieur, un hydroxy-alkyle inférieur ou un carboxy-alkyle inférieur, Z est un alcanoyle en C₆ à C₁₈, ou bien Z, conjointement avec R₁ et R₂, constitue un carbone substitué par un alkyle en C₅ à C₁₇, et n et m représentent chacun de 1 à 4, en association avec suffisamment d'ions de charge contraire pour se traduire par une charge nette du composé de zéro; ledit agent réglant le pH est choisi parmi l'acide chlorhydrique ou lactique; et ledit agent améliorant la viscosité est choisi parmi le poloxamère 407 et le ploxamère 101.

6. La solution de la revendication 3, dans laquelle l'alcanol en C₂ à C₆ est de l'éthanol ou de l'isopropanol; ladite charge de tonicité est le chlorure de sodium; ledit agent tensioactif correspond à la formule 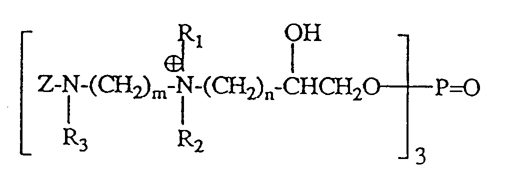 dans laquelle chacune des R₁ et R₂ représente de façon indépendante, un alkyle inférieur, un hydroxy-alkyle inférieur ou un carboxy-alkyle inférieur, R₃ est de l'hydrogène, un alkyle inférieur, un hydroxy-alkyle inférieur ou un carboxy-alkyle inférieur, Z est un alcanoyle en C₆ à C₁₈, ou bien Z, conjointement avec R₁ et R₂, constitue un carbone substitué par un alkyle en C₅ à C₁₇, et n et m représentent chacun de 1 à 4, en association avec suffisamment d'ions de charge contraire pour se traduire par une charge nette du composé de zéro; ledit agent réglant le pH est choisi parmi l'acide chlorhydrique ou lactique; et ledit agent améliorant la viscosité est choisi parmi le poloxamère 407 et le poloxamère 101.

7. La solution de la revendication 5 dans laquelle ledit agent tensioactif correspond à la formule

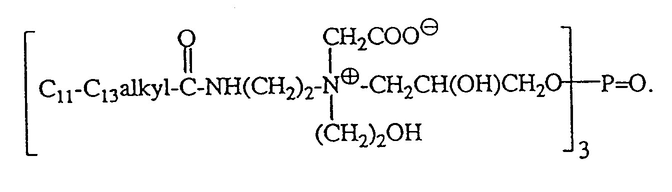

8. La solution de la revendication 1, dans laquelle ledit alkylène en C₃ à C₈ glycol est le propylène glycol; ledit alcanol inférieur est de l'alcool isopropylique; ladite charge de tonicité est du chlorure de sodium; ledit agent tensioactif correspond à la formule 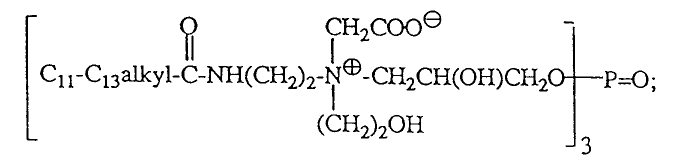 ledit agent d'ajustement ou de réglage du pH est l'acide lactique; ledit agent améliorant la viscosité est de l'hydroxy-éthyl cellulose.

9. La solution de la revendication 1, dans laquelle ledit alkylène en C₃ à C₈ glycol est présent en une quantité de 21% en poids; ledit alcanol inférieur est présent en une quantité de 16% en poids; ledit agent tensioactif est présent en une quantité de 5% en poids; ledit agent d'ajustement ou de régulation du pH est présent en une quantité de 1,1% en poids; ladite charge de tonicité est présente en une quantité équivalente à une solution à 12,5% de chlorure de sodium; et ledit agent améliorant la viscosité est présent en une quantité de 0,1% en poids.

10. La solution de la revendication 1, comportant de 0 à 30% d'hexylène glycol, de 15 à 20% d'isopropanol, de 7 à 12% de chlorure de sodium, de 5 à 10% de Miranol, de 5 à 15% de poloxamère 407 ou 101 et d'eau.

11. Un procédé de désinfection et de nettoyage d'une lentille de contact, qui consiste à appliquer par frottement sur la surface de ladite lentille de contact une quantité désinfectante et nettoyante, efficace d'une solution de la revendication 1 pendant une période de 5 à 30 secondes, suivi par un rinçage de ladite lentille de contact avec de l'eau ou une solution saline normale.

12. Le procédé de la revendication 11, dans lequel sur chaque face de ladite lentille de contact, est appliquée par frottement ladite solution pendant 15 secondes après quoi la lentille de contact en totalité est rincée avec une solution saline normale pendant 10 secondes.

13. Le procédé de la revendication 12, dans lequel l'étape de rinçage est suivie par un séjour de ladite lentille de contact dans une solution saline normale pendant 1 minute.

14. L'utilisation d'une solution selon l'une quelconque des revendications 1 à 10 pour désinfecter et nettoyer les lentilles de contact.

15. Procédé de fabrication d'une solution selon l'une quelconque des revendications 1 à 10, caractérisé par un mélange approprié des composants individuels.

## Revendications (Revendications pour l'(les) Etat(s) contractant(s) suivant(s): ES)

1. Un procédé de fabrication d'une solution pour nettoyer et désinfecter des lentilles de contact comportant
a) x % en poids d'un alkylène (en C₃ à C₈) glycol et
y % en poids d'un alcanol en C₂ à C₆,
x étant de zéro à 50 tandis que y est de zéro à 30 sous la condition que $\text{x/10 + y/2 ≧ 1,0}$;
b) une quantité d'une charge de tonicité, suffisante pour amener la tonicité de la solution à l'équivalent d'une solution de chlorure de sodium de 5 à 20%;
c) de 2 à 25% en poids d'un agent tensioactif compatible avec le matériau constitutif du dispositif ophtalmique;
d) de 0 à 2% en poids d'un agent d'ajustement ou de régulation du pH;
e) de 0 % à une quantité suffisante pour amener la viscosité de la solution à 100 cps d'un agent améliorant la viscosité; et
f) le reste étant un solvant approprié, qui est de préférence de l'eau,
caractérisé par un mélange approprié des composants individuels.

2. Le procédé de la revendication 1, comportant le mélange de
a) de 10 à 50% en poids d'un alkylène (en C₃ à C₈) glycol et de 2 à 30% en poids d'un alcanol en C₂ à C₆.
b) une quantité d'un halogénure, d'un phosphate, d'un carbonate ou d'un sulfate de métal alcalin ou de métal alcalino terreux, acceptable du point de vue ophtalmique qui est suffisante pour amener la tonicité de la solution à celle d'une solution de 5 à 20% en poids de chlorure de sodium;
c) de 2 à 15% d'un agent tensioactif compatible avec un matériau constitutif d'un dispositif ophtalmique, choisi parmi:
ca) les composés de formule I
(AmSur-O)₃-P=O (I)
dans laquelle le groupe AmSur correspond à la formule 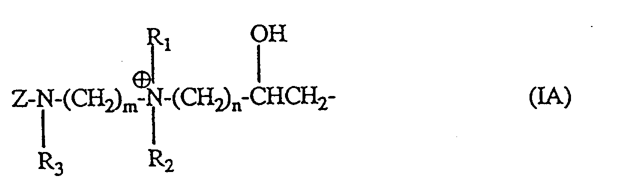 dans laquelle chacun des R₁ et R₂ représente, de façon indépendante, un radical alkyle inférieur, hydroxy-alkyle inférieur ou carboxy-alkyle inférieur, R₃ est de l'hydrogène, un groupe alkyle inférieur, hydroxy-alkyle inférieur ou carboxyl-alkyle inférieur, Z est un alcanoyle de 6 à 18 atomes de carbone ou bien Z conjointement avec R₁ et R₂ représente un carbone substitué par un alkyle en C₅ à C₁₇; et n et m représentent chacun de façon indépendante, de 1 à 4;
cb) composés de la formule 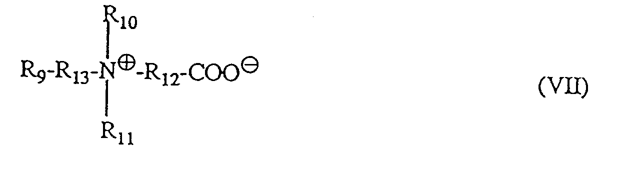 dans laquelle R₉ est un alkyle de 5 à 17 atomes de carbone ou un alcanoyle (en C₆ à C₂₀) amino; chacun des R₁₀ et R₁₁ représentent indépendamment un radical alkyle inférieur, hydroxyl-alkyle inférieur ou carboxy-alkyle inférieur; R₁₂ est un alpha,oméga-alkylène de 1 à 6 atomes de carbone qui est non-substitué ou substitué par un groupe alkyle inférieur, hydroxy ou hydroxy-alkyle inférieur et R₁₃ est un alpha, oméga-alkylène en C₁ à C₅;
cc) composés de la formule 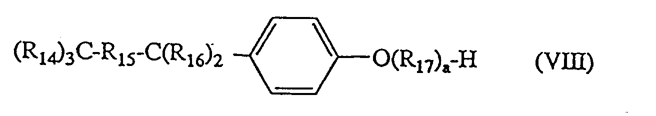 dans laquelle chaque R₁₄ et chaque R₁₆ représentent indépendamment un alkyle en C₁ à C₄; R₁₅ est un alpha,oméga-alkyle en C₁ à C₄; chaque R₁₇ représente indépendamment -CH₂CH₂O-, -CH₂CH₂CH₂O-, ou -CH₂CH(CH₃)-O-et a est de 3 à 18; et
cd) composés de la formule 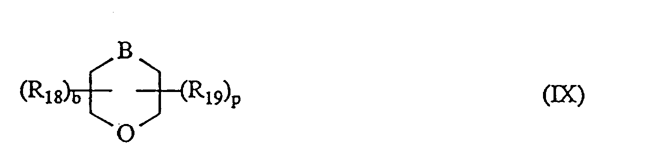 dans laquelle B est un alpha,oméga-alkylène en C₁ à C₄; p est un nombre entier de 0 à (d-1); b est un nombre entier qui est (d-p-1); d est de 4 à 7; chaque R₁₈ représente indépendamment H ou un alkyle en C₁ à C₄ qui est non-substitué ou substitué par au moins un R₁₉; chaque R₁₉ représente indépendamment un radical hydroxy qui est libre, éthérifié par R₂₀ ou bien estérifié par R₂₁; chaque R₂₀ est un oxyalkylène à chaîne droite ou ramifiée en C₂ à C₄ ou un poly(oxyalkylène à chaîne droite ou ramifiée en C₁ à C₄), dont l'oxygène terminal est lié à H ou R₂₁; et chaque R₂₁ représente indépendamment un acyle d'un acide alcanoïque en C₂ à C₂₄ ou d'un acide alcénoïque en C₄ à C₂₄, sous la condition que dans chaque groupe de formule IX, il existe au moins un groupe hydroxyle libre et au moins un groupe R₂₁;
ou bien les poloxyamères, les poloxyamères inversés, les méroxapols, les poloxamines, les polyéthylèneglycols, les polypropylèneglycols, les propylèneglycol-buteths, les oléates de polypropylèneglycol, les polypropylène-pareths, la tétrahydroxypropyl-éthylènediamine, les cétéareths, les sels NTA, les sels d'EDTA et les sels de pentétate;
d) de 0 à 2% en poids d'un agent d'ajustement ou de réglage du pH choisi parmi i) l'acide phosphorique, l'acide borique, l'acide lactique et l'acide citrique, ii) un sel acceptable du point de vue ophtalmique de ceux-ci, iii) un mélange dudit acide et d'un sel dudit acide, iv) un acide inorganique acceptable du point de vue ophtalmique et v) d'une base inorganique acceptable du point de vue ophtalmique;
e) de 0 % à une quantité suffisante pour amener la viscosité de la solution à 100 cps d'un agent améliorant la viscosité choisi parmi les hydroxy-alkyl inférieur-celluloses, les hydroxy alcanoyl inférieur-celluloses, les alkyl inférieur-celluloses, les alcanoyl inférieur-celluloses, les carboxy-alkyl inférieur-celluloses, les polaxamères, les polyéthylèneglycols, les polypropylèneglycols, les propylèneglycol-buteths, les oléates de polypropylèneglycol, les polypropylène-pareths, la tétrahydroxypropyl-éthylènediamine, les cétéareths, les sels NTA, les sels d'EDTA et les sels de pentétate; et
f) de l'eau;
la dénomination "inférieur" se rapportant aux résidus ayant jusqu'à 7 atomes de carbone.

3. Le procédé de la revendication 2, dans lequel l'alkylène glycol est omis.

4. Le procédé de la revendication 1, dans lequel ledit alkylène en C₃ à C₈ glycol est du propylène glycol ou de l'hexylène glycol; ledit alcanol en C₂ à C₆ est de l'éthanol ou de l'isopropanol; ladite charge de tonicité est un halogénure, un phosphate, un carbonate ou un sulfate de métal alcalin ou alcalino-terreux, acceptable du point de vue ophtalmique;
ledit agent tensioactif correspond à la formule: 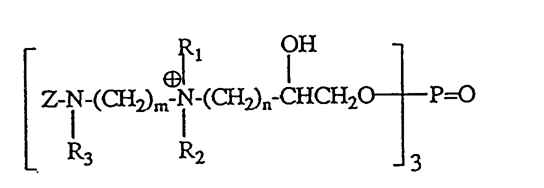 dans laquelle chacune des R₁ et R₂ représente de façon indépendante, un alkyle inférieur, un hydroxy-alkyle inférieur ou un carboxy-alkyle inférieur, R₃ est de l'hydrogène, un alkyle inférieur, un hydroxy-alkyle inférieur ou un carboxy-alkyle inférieur, Z est un alcanoyle en C₆ à C₁₈, ou bien Z, conjointement avec R₁ et R₂, constitue un carbone substitué par un alkyle en C₅ à C₁₇, et n et m représentent chacun de 1 à 4, en association avec suffisamment d'ions de charge contraire pour se traduire par une charge nette du composé de zéro; ledit agent d'ajustement ou de réglage du pH est choisi parmi i) l'acide phosphorique, l'acide borique, l'acide lactique et l'acide citrique, ii) un sel acceptable du point de vue ophtalmique de ceux-ci, iii) un mélange dudit acide et d'un sel dudit acide, iv) un acide inorganique acceptable du point de vue ophtalmique et v) d'une base inorganique acceptable du point de vue ophtalmique; ledit agent améliorant la viscosité est choisi parmi les hydroxy-alkyl inférieurs-cellulose, les hydroxy-alcanoyl inférieurs-cellulose, les alkyl inférieurs-cellulose, les alcanoyl inférieurs-cellulose et les carboxy-alkyl inférieurs-cellulose.

5. Le procédé de la revendication 2, dans lequel l'alkylène glycol en C₃ à C₈ est du propylène glycol ou de l'hexylène glycol, ledit alcanol en C₂ à C₆ est de l'éthanol ou de l'isopropanol; ladite charge de tonicité est du chlorure de sodium;
ledit agent tensioactif correspondant à la formule: 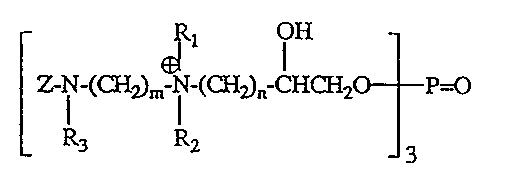 dans laquelle chacune des R₁ et R₂ représente de façon indépendante, un alkyle inférieur, un hydroxy-alkyle inférieur ou un carboxy-alkyle inférieur, R₃ est de l'hydrogène, un alkyle inférieur, un hydroxy-alkyle inférieur ou un carboxy-alkyle inférieur, Z est un alcanoyle en C₆ à C₁₈, ou bien Z, conjointement avec R₁ et R₂, constitue un carbone substitué par un alkyle en C₅ à C₁₇, et n et m représentent chacun de 1 à 4, en association avec suffisamment d'ions de charge contraire pour se traduire par une charge nette du composé de zéro; ledit agent réglant le pH est choisi parmi l'acide chlorhydrique ou lactique; et ledit agent améliorant la viscosité est choisi parmi le poloxamère 407 et le ploxamère 101.

6. Le procédé de la revendication 3, dans lequel l'alcanol en C₂ à C₆ est de l'éthanol ou de l'isopropanol; ladite charge de tonicité est le chlorure de sodium; ledit agent tensioactif correspond à la formule 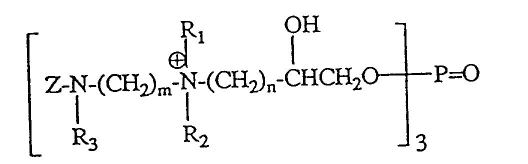 dans laquelle chacune des R₁ et R₂ représente de façon indépendante, un alkyle inférieur, un hydroxy-alkyle inférieur ou un carboxy-alkyle inférieur, R₃ est de l'hydrogène, un alkyle inférieur, un hydroxy-alkyle inférieur ou un carboxy-alkyle inférieur, Z est un alcanoyle en C₆ à C₁₈, ou bien Z, conjointement avec R₁ et R₂, constitue un carbone substitué par un alkyle en C₅ à C₁₇, et n et m représentent chacun de 1 à 4, en association avec suffisamment d'ions de charge contraire pour se traduire par une charge nette du composé de zéro; ledit agent réglant le pH est choisi parmi l'acide chlorhydrique ou lactique; et ledit agent améliorant la viscosité est choisi parmi le poloxamère 407 et le poloxamère 101.

7. Le procédé de la revendication 5 dans lequel ledit agent tensioactif correspond à la formule

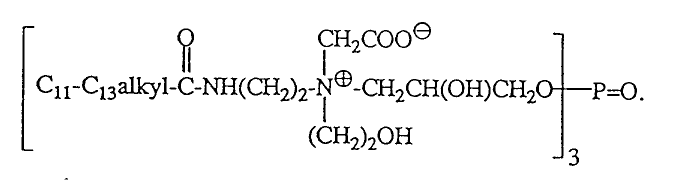

8. Le procédé de la revendication 1, dans lequel ledit alkylène en C₃ à C₀ glycol est le propylène glycol; ledit alcanol inférieur est de l'alcool isopropylique; ladite charge de tonicité est du chlorure de sodium; ledit agent tensioactif correspond à la formule 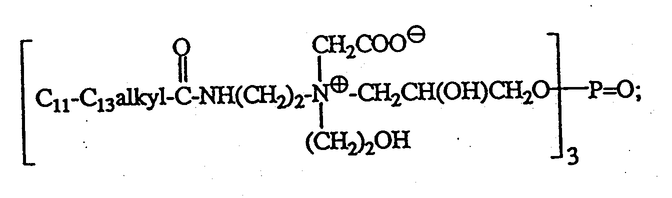 ledit agent d'ajustement ou de réglage du pH est l'acide lactique; ledit agent améliorant la viscosité est de l'hydroxy-éthyl cellulose.

9. Le procédé de la revendication 1, dans lequel ledit alkylène en C₃ à C₈ glycol est présent en une quantité de 21% en poids; ledit alcanol inférieur est présent en une quantité de 16% en poids; ledit agent tensioactif est présent en une quantité de 5% en poids; ledit agent d'ajustement ou de régulation du pH est présent en une quantité de 1,1% en poids; ladite charge de tonicité est présente en une quantité équivalente à une solution à 12,5% de chlorure de sodium; et ledit agent améliorant la viscosité est présent en une quantité de 0,1% en poids.

10. Le procédé de la revendication 1, comportant le mélange de 0 à 30% d'hexylène glycol, de 15 à 20% d'isopropanol, de 7 à 12% de chlorure de sodium, de 5 à 10% de Miranol, de 5 à 15% de poloxamère 407 ou 101 et d'eau.

11. Un procédé de désinfection et de nettoyage d'une lentille de contact, qui consiste à appliquer par frottement sur la surface de ladite lentille de contact une quantité désinfectante et nettoyante, efficace d'une solution de la revendication 1 pendant une période de 5 à 30 secondes, suivi par un rinçage de ladite lentille de contact avec de l'eau ou une solution saline normale.

12. Le procédé de la revendication 11, dans lequel sur chaque face de ladite lentille de contact, est appliquée par frottement ladite solution pendant 15 secondes après quoi la lentille de contact en totalité est rincée avec une solution saline normale pendant 10 secondes.

13. Le procédé de la revendication 12, dans lequel l'étape de rinçage est suivie par un séjour de ladite lentille de contact dans une solution saline normale pendant 1 minute.

14. L'utilisation d'une solution selon l'une quelconque des revendications 1 à 10 pour désinfecter et nettoyer les lentilles de contact.
